# NEW EUROPEAN PATENT SPECIFICATION

(11) **EP 3 663 890 B2**
(45) Date of publication and mention of the opposition decision: **02.12.2026**
(45) Mention of the grant of the patent: 10.04.2024
(21) Application number: 17919940.1
(22) Date of filing: 02.08.2017
(51) Int. Cl.: G16B 30/10, G16B 30/20, G16B 35/10

(54) **ALIGNMENT METHOD, DEVICE AND SYSTEM**
AUSRICHTVERFAHREN, -VORRICHTUNG UND -SYSTEM
PROCÉDÉ, DISPOSITIF ET SYSTÈME D'ALIGNEMENT

(43) Date of publication of application: 10.06.2020
(73) Proprietor: GeneMind Biosciences Company Limited, Shenzhen City, Guangdong 518023 (CN)
(72) Inventor: XU, Weibin, Shenzhen City, 518023 Guangdong (CN); JIN, Huan, Shenzhen City, 518023 Guangdong (CN); YAN, Qin, Shenzhen City, 518023 Guangdong (CN); JIANG, Zefei, Shenzhen City, 518023 Guangdong (CN); ZHOU, Zhiliang, Shenzhen City, 518023 Guangdong (CN)
(74) Representative: Schiweck Weinzierl Koch Patentanwälte Partnerschaft mbB
(86) International application number: PCT/CN2017/095612
(87) International publication number: WO 2019/023978

(56) References cited:
- CN-A- 103 294 932
- CN-A- 103 451 279
- CN-A- 105 243 297
- US-A1- 2011 257 889
- US-A1- 2012 089 338
- US-A1- 2012 089 338
- US-A1- 2014 114 584
- HONGYI XIN ET AL: "Accelerating read mapping with FastHASH", BMC GENOMICS, BIOMED CENTRAL, vol. 14, no. Suppl 1, 21 January 2013 (2013-01-21), pages S13, XP021134838, ISSN: 1471-2164, DOI: 10.1186/1471-2164-14-S1-S13
- RIZK, G. ET AL.: "GASSST: global alignment short sequence search tool", BIOINFORMATICS, vol. 26, no. 20, 15 October 2010 (2010-10-15), pages 2534 - 2540, XP055567315

## Description

### TECHNICAL FIELD

The present invention relates to the field of data processing, and more particularly relates to a sequence alignment method, a sequence alignment device, and a sequence alignment system.

### BACKGROUND

In biological data processing and analysis, alignment, as a basic part of biological information analysis, puts forward certain requirements on time-consuming and efficiency, and different alignment models and algorithms have different sensitivity and accuracy.

At present, mainstream alignment software, such as Bwa, Bowtie, and SOAP, is mainly developed for sequences generated by the second generation sequencing platform, while the software such as Blast and Mapq is mainly developed for long sequences. A read mapping algorithm has also been proposed e.g. by Xin et al. (BMC Genomics 14 (Suppl 1), S13 (2013), https://doi.org/10.1186/1471-2164-14-S1-S13). US 2012/089338 A1 relates to methods for indexing a reference genome, and US 2014/114584 A1 is directed to automated processing of read symbol sequences to identify variations between a sequence assembled from overlapping read sequences and a reference symbol sequence.

Current alignment methods need to be improved in terms of sensitivity, time-consuming and/or alignment efficiency of specific types of data.

### SUMMARY

The embodiments of the present invention aim to at least solve one of the technical problems existing in the prior art or to at least provide a commercial means.

According to an aspect of the present invention a computer-implemented alignment method according to claim 1 is provided.

According to another aspect of the present invention, an alignment device according to claim 8 is provided, preferably for implementing all or part of the steps of the aforementioned alignment method.

According to yet another aspect of the present invention, a computer readable medium is provided for storing/carrying a computer executable program. It will be understood by those skilled in the art that when the program is executed, all or part of the steps of the aforementioned alignment method can be performed by instructing related hardware. The medium may include: a read-only memory, a random access memory, a magnetic disk or an optical disk, and the like.

According to still another aspect of the present invention, an alignment system is provided, which includes an input device configured to input data; an output device configured to output data; a processor configured to execute a computer executable program, wherein the executing the computer executable program includes performing the aforementioned computer-implemented alignment method of the present invention; and a storage device configured to store data, wherein the storage device includes the computer executable program.

The invention is defined in the appended claims. Embodiments of the invention are the subject-matter of the dependent claims and are disclosed throughout the present description and figures. The alignment method, device and/or system in accordance with the present invention have higher sensitivity and accuracy by converting a read into short fragments and converting the sequence information into position information, and can efficiently and accurately process the off-line data of various sequencing platforms, including the off-line data of long-read sequencing and short-read sequencing. In particular, it is particularly suitable for efficient and accurate positioning of reads containing unrecognized bases, i.e., reads containing gaps.

Additional aspects and advantages of the present invention will be set forth in part in the following description, which will become apparent from the following description, or may be appreciated by practice of the present invention.

### BRIEF DESCRIPTION OF THE DRAWINGS

The above and/or additional aspects and advantages of the present invention will become apparent and readily appreciated from the following description of the embodiments, taken in conjunction with the accompanying drawings of which:
FIG. 1 is a flow chart of an alignment method according to an embodiment of the present invention.
FIG. 2 is a schematic view of a distance between two entries of a reference library.
FIG. 3 is a schematic view of a connectivity length according to an embodiment of the present invention.
FIG. 4 is a schematic view of an alignment device according to an embodiment of the present invention.
FIG. 5 is a schematic view of an alignment device according to an embodiment of the present invention.
FIG. 6 is a schematic view of an alignment device according to an embodiment of the present invention.
FIG. 7 is a schematic view of an alignment device according to an embodiment of the present invention.
FIG. 9 is a schematic view of an alignment device according to an embodiment of the present invention.

### DETAILED DESCRIPTION OF THE EMBODIMENTS

Embodiments of the present invention are described in detail below, and examples of the embodiments are illustrated in the drawings, wherein identical or similar reference numerals represent identical or similar elements or elements having identical or similar functions. The embodiments described below with reference to the accompanying drawings are exemplary and are intended to be illustrative of the invention and not to be construed as limiting.

In this description, the terms "first" and "second" are used for descriptive purposes only, and are not to be construed as indicating or implying relative importance and/or order.

The terms "connected" and "coupled" used herein are to be understood broadly, and may be, for example, fixedly connected, detachably connected, or integrally connected; can be mechanically or electrically connected; may be connected directly or indirectly through intervening media, or may be internal communication between two elements. The specific meaning of the above terms herein can be understood in specific circumstances by those skilled in the art.

The term "read" used herein refers to a sequence fragment obtained by sequencing a DNA/RNA/protein sequence, including a sequence obtained by determining and identifying at least a portion of a DNA/RNA/protein sequence using a sequencing platform. The sequencing platform can be selected from, but not limited to, Hisq/Miseq/Nextseq sequencing platform of Illumina, Ion Torrent platform of Thermo Fisher (Life Technologies), BGISEQ platform of BGI, and the single-molecule sequencing platform. The sequencing mode can be either single-end sequencing or paired-end sequencing, and the obtained off-line data can be sequencing and reading fragments called reads.

The term "alignment" used herein refers to a sequence alignment, including the process of positioning a read to a reference sequence, as well as the process of obtaining a read position/match(mapping) result.

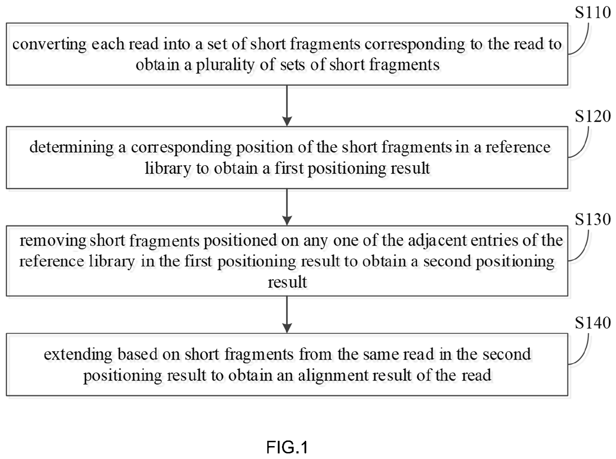

The present invention relates to a computer-implemented alignment method according to claim 1. Referring to FIG. 1, an alignment method according to an embodiment of the present invention accordingly includes the following steps: S110, each read is converted into a set of short fragments of length *L* corresponding to the respective read to obtain a plurality of sets of short fragments; S 120, a corresponding position of the short fragments in a reference library is determined by aligning the short fragments with the reference library to obtain a first positioning result, the reference library is a hash table constructed based on a reference sequence, the reference library includes a plurality of entries, each entry of the reference library corresponds to a seed sequence of length *L*, and the seed sequence is configured to match at least one sequence on the reference sequence, and a distance on the reference sequence between two seed sequences corresponding to two adjacent entries of the reference library is less than the length of the short fragments; per read: S 130, short fragments positioned on an one of the adjacent entries of the reference library in the first positioning result are removed to obtain a second positioning result; and, S 140, an extension is performed based on short fragments from the same read in the second positioning result to obtain an alignment result of the respective read. By converting the read into the short fragments and converting the read sequence information into the position information, that is, converting the sequence form into a digital form, the computer-implemented alignment method according to the present invention is favorable for quickly and accurately achieving the alignment and positioning of the off-line data of various sequencing platforms. In particular, it is particularly suitable for quick and accurate alignment of reads containing unrecognized bases, i.e., reads containing gaps or N, such as alignment analysis of reads obtained due to poor sequencing quality, poor base recognition, and the like.

The reference sequence (ref) used herein may be a predetermined sequence, which may be a DNA and/or RNA sequence previously assembled by itself, may be a DNA and/or RNA sequence determined and disclosed by others, and may be any previously obtained reference template in a biological category to which a target individual belongs, for example, all or at least a portion of a disclosed genome assembly sequence of the same biological category. If the target individual is a human, its genome reference sequence (also referred to as the reference genome or reference chromosomes) may be selected from HG19 provided by the NCBI database. In addition, a resource library containing more reference sequences can be configured in advance, and before sequence alignment, a closer sequence may be selected or determined and assembled as a reference sequence according to factors such as sex, race, region, and the like of the target individual, so that more accurate sequence analysis results can be obtained subsequently. The reference sequence may contain the chromosome number and the position information of each site on the chromosome. The reference library used herein is a hash table, and the reference library may be built by directly using the seed sequence used herein as a key (key name) and using the position of the seed sequence used herein on the reference sequence as a value (key value). It is also possible to first convert the seed sequence used herein into a numeric or integer string with the numeric or integer string as the key and the position of the seed sequence on the reference sequence as the value to establish the reference library. The position of the seed sequence on the reference sequence as the value used herein may be the corresponding one or more positions of the seed sequence on the reference sequence/chromosome. The position may be represented directly by a real numerical value or a range of numerical values, and may also be re-encoded to be represented by a custom character and/or number. According to an embodiment of the present invention, the construction of the hash table by using a vector of C++ may be expressed as follows: *Hash*(*seed*) = *Vector*(*position*). The vector used herein may be an object entity that can hold many other types of the same element, and may therefore also be referred to as a container. The reference library can be built by saving in binary. In addition, the hash table may be divided into blocks for storage, and a block head key and a block tail key are set in the block head. For example, for sequential sequence block {5, 6, 7, 8..., 19, 20}, the block head and the block tail (or a table head and a table tail) 5 and 20 are set, if there is a number of 3, since 3<5, it can be seen that 3 does not belong to the sequential sequence block, and if there is a number of 10, since 5<10<20, it is known that 10 belongs to the sequential sequence block. In this way, the global index can be selected during query, and the block where the global index is located can be quickly located by comparing the block head key and the block tail key without the need of the global index.

The reference library used herein may be built when the sequence is to be aligned, or may be built and stored in advance. According to a specific embodiment of the present invention, the reference library is pre-built and stored for use. According to an embodiment of the present invention, the building of the reference library includes: a length *L* of the seed sequence is determined according to total number of bases *totalBase* of the reference sequence, *L* = *µ* * *log*(*totalBase*), *µ* ⊂ [1,3] and *L* may be less than a length of a read to be aligned and analyzed (read length); all possible seed sequences are generated based on the length of the seed sequence to obtain a seed sequence set; and a seed sequence capable of matching the reference sequence in the seed sequence set and a matching position of the seed sequence are determined to obtain the reference library. Based on the relationship between the length of the seed sequence and the reference sequence established by the inventor through multiple hypothesis test verification, the method enables the built reference library to contain a comprehensive seed sequences with the correlation information of the corresponding positions of the seed sequences on the reference sequence. The reference library may have a compact structure and small memory occupation and can be used for high-speed access query in sequence positioning analysis. An entry of the reference library obtained according to this embodiment may contain only one key, one key corresponds to at least one value.

This embodiment of the present invention is not limited to the method of generating all possible seed sequences and obtaining the seed sequence set. For inputting a set, elements in the set may be traversed to obtain all possible combinations of elements of a specific length, which may be implemented by using a recursive algorithm and/or a loop algorithm, for example.

In one example, the reference sequence is a human genome containing about 3 billion bases, the length of the reads to be processed is not less than 25 bp, and *L* takes an integer in [11, 15] to facilitate efficient alignment.

In one example, the reference sequence is a human cDNA reference genome, the total number of bases *totalBase* of the reference genome is counted, and the length *L* of the seed sequence (seed) is set based on the total number of bases, *L* (*seed*) = *log* (*totalBase*) * *µ, µ* ⊂ [1,3] . Based on the length *L* and the base types of the DNA sequences including A, T, C, and G, a set of all possible seed sequences is generated by using a recursive algorithm to obtain the seed sequence set, which can be represented as *seed* = *B*₁*B*₂...*B_{L},B* ∈ {*ATCG*}. The seed sequence capable of matching the reference genome in the seed sequence set and the matching position of the seed sequence are determined to obtain the reference library, so that the reference library is built and obtained by taking the seed sequences capable of matching the reference genome as the key and the position of the seed sequence on the reference genome as the value.

In one example, the reference sequence is the DNA genome and transcriptome of a species, the total number of bases *totalBase* of the reference sequence is counted, and the length *L* of the seed sequence (seed) is set based on the total number of bases, *L* (*seed*) = *log* (*totalBase*) * *µ, µ* ⊂ [1,3] . Based on the length *L*, the base types constituting the DNA sequences including A, T, C, and G, and the base types constituting the RNA sequences including A, U, C, and G, a set of all possible seed sequences is generated by using a recursive algorithm to obtain the seed sequence set, which can be represented as *seed* = *B*₁*B*₂...*B_{L}*, *B* ∈ {*ATCG*} ∪ {*AUCG*}. The seed sequence capable of matching the reference sequence in the seed sequence set and the matching position of the seed sequence are determined to obtain the reference library, so that the reference library is built and obtained by taking the seed sequences capable of matching the reference sequence as the key and the position of the seed sequence on the reference sequence as the value.

In one example, the seed sequence can be converted into a string consisting of numeric characters, and the string is used as a key to build a library, so that the speed of accessing the reference library built by the query can be improved. For example, after a seed sequence capable of matching the reference sequence is obtained, the seed sequence is encoded as follows: ${seed}_{i} = \left\{\begin{matrix}00 & i = A \\ 01 & i = T or U \\ 10 & i = C \\ 11 & i = G\end{matrix}\right.$. For example, after the seed sequence set is obtained, the seed sequence in the seed sequence set is encoded, and the base encoding rule may be the same as described above. In addition, the encoding conversion of the same rule may be carried out on the reference sequence, which is favorable for quickly obtaining the corresponding position information of the seed sequences on the reference sequence and improving the speed of accessing and querying the built reference library.

According to a specific embodiment of the present invention, the determining the seed sequence capable of matching the reference sequence in the seed sequence set and the matching position of the seed sequence includes: a sliding window is performed on the reference sequence by using a window having a size of *L*, the seed sequence in the seed sequence set is matched with a window sequence obtained by the sliding window to determine the seed sequence capable of matching the reference sequence in the seed sequence set and the matching position of the seed sequence, and an error tolerance rate for the matching is *ε*₁, to obtain the reference library. In this way, the corresponding position information of the seed sequence on the reference sequence can be obtained quickly, which facilitates quickly building and obtaining the reference library. The error tolerance rate used herein may be a proportion of allowable mismatched bases, and the mismatch may be at least one selected from the group consisting of substitution, insertion, and deletion.

In one example, the match used herein is a strict matching, that is, the error tolerance rate *ε*₁ is zero, and when the seed sequence is exactly identical to one or more sliding window sequences, the position of the sliding window sequence is the corresponding position of the seed sequence on the reference sequence. In another example, the match used herein is an error-tolerant matching, and the error tolerance rate *ε*₁ is greater than zero. When the proportion of the inconsistency of the bases at the same positions of the seed sequence and one or more sliding window sequences is less than the error tolerance rate, the position of the sliding window sequence is the corresponding position of the seed sequence on the reference sequence. In one example, the corresponding position of the seed sequence on the reference sequence is encoded, and the reference library is built with the encoded characters, such as numeric characters, as values.

In other words, the error tolerance rate *ε*₁ may not be zero, which is equivalent to converting a seed sequence into a group of seed templates allowed by *ε*₁. For example, if seed = ATCG, and *ε*₁ is 0.25, that is, one error is allowed in four bases, then the seed template can be ATCG, TTCG, CTCG, GTCG, AACG, ACCG, AGCG, and the like. When the position of seed = ATCG on the reference sequence is determined at the error tolerance rate of 0.25, it is equivalent to determining the positions of all seed templates corresponding to the seed on the reference sequence, for example, ref = ATCG, all the previously shown seed templates may be matched to this position, and ref = TTCG, seed templates ATCG, TTCG, CTCG, or GTCG may be matched to the position. Furthermore, the built reference library may use a seed as a key, or each of all seed templates corresponding to the seed as a key, where the key is different from the key, and one key corresponds to at least one value.

According to a specific embodiment of the present invention, when determining the corresponding position of the seed sequence on the reference sequence, a step size of performing the sliding window on the reference sequence is determined in accordance with *L* and *ε*₁. In one example, the step size of performing the sliding window is no less than *L* * *ε*₁. In a specific example, the reference sequence is a human genome containing about 3 billion bases, the length of the reads to be processed is not less than 25 bp, *L* is 14 bp, and *ε*₁ is 0.2-0.3, the step size of performing the sliding window is 3 bp to5 bp, so that two adjacent windows can stride over continuous error combination under the condition of *ε*₁ in the positioning process of the sliding window, and rapid positioning is facilitated. In one example, a distance between two adjacent entries of the built reference library is the step size of the sliding window.

According to a specific embodiment of the present invention, S 110 includes: a sliding window is performed on the read by using a window having a size of *L* to obtain a set of short fragments corresponding to the read, and a step size of performing the sliding window is 1 bp. In this way, for a piece of reads with a length of K, (K-L + 1) pieces of short fragments with a length of *L* are obtained, the reads are converted into the short fragments, the corresponding position of each short fragment in the reference library is determined by using high-speed access to query the reference library, thereby obtaining the information of the reads corresponding to the short fragments in the reference library.

According to a specific embodiment of the present invention, S 120 includes: the short fragments are matched with seed sequences corresponding to entries of the reference library to determine the positions of the short fragments in the reference library, and an error tolerance rate for the matching is *ε*₂.

In one example, the match used herein is a strict matching, that is, the error tolerance rate *ε*₂ is zero. When a short fragment is exactly identical to a seed or a seed template corresponding to an entry of the reference library, the position information of the short sequence on the reference library is obtained. In another example, the match used herein is an error-tolerant matching, and the error tolerance rate *ε*₂ is greater than zero. The position information of the short sequence on the reference library is obtained when the proportion of bases in which the short sequence does not match the seed or the seed template corresponding to one or more entries of the reference library is less than the error tolerance rate. In a specific example, *ε*₂ = *ε*₁ and not zero, enabling more valid data to be obtained.

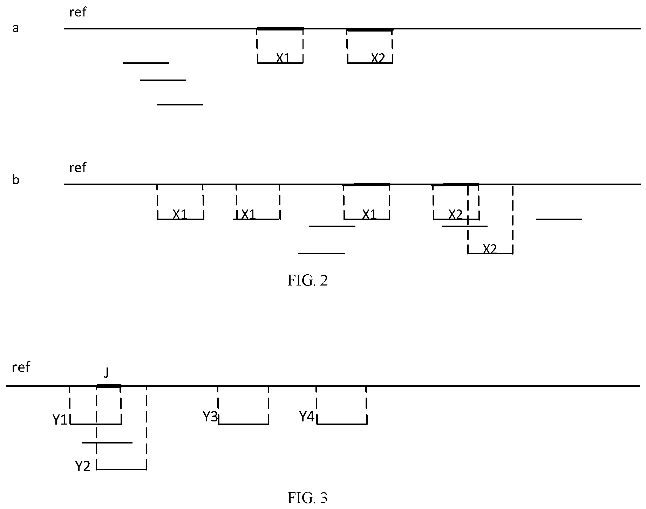

Referring to FIG. 2 (it is to be noted that solely for illustrative purposes of principles of determining a distance on the reference sequence between two seed sequences that correspond to two adjacent entries of the reference library, the seed sequences X1 and X2 shown in Fig. 2 are depicted as non-overlapping on the reference sequence; however, in the context of the present invention a distance on the reference sequence between two seed sequences of length *L* that correspond to two adjacent entries of the reference library is less than the length *L* of the short fragments), at S120, the distance on the reference sequence between the two seed sequences X1 and X2 corresponding to two adjacent entries of the reference library may be divided into the following two cases. When the keys and values of the two entries of the reference library are both unique, that is, one entry corresponds to one [key, value], and referring to FIG. 2a, which is equivalent to when X1 and X2 are uniquely matched with the reference sequence (both X1 and X2 match only one position of the reference sequence), the distance used herein may be a distance between the two positions corresponding to X1 and X2 on the reference sequence, and the two positions are shown in the bold black lines. When the key of at least one of the two entries of the reference library corresponds to multiple values, referring to FIG. 2b, which is equivalent to that at least one of the two seed sequences X1 and X2 and the reference sequence are non-uniquely matched, that is, at least one of X1 and X2 matches multiple positions of the reference sequence, the distance used herein may be a distance between the two closest positions on the reference sequence corresponding to the X1 and X2, and the two positions are shown in bold black lines. The method of expressing the distance between two sequences is not limited, and may be expressed as, for example, a distance from any one of the both ends of one sequence to any one of the ends of the other sequence, or a distance from the center of one sequence to the center of the other sequence.

According to a specific embodiment of the present invention, after the second positioning result is obtained, S130 further includes: short fragments having a connectivity length less than a predetermined threshold are removed, and the second positioning result is replaced with a removed result, wherein the connectivity length is a total length of short fragments in the second positioning result (which is herein also referred to as second alignment result) that are from the (same) read and are positioned to different entries of the reference library mapped to the reference sequence. This process facilitates the removal of some data that is transitionally redundant and/or of relatively low quality, facilitating an increase in speed of alignment.

The connectivity length may be expressed as a sum of the lengths of the short fragments from the (same) read and positioned to different entries of the reference library minus the length of the overlapping portion of the short fragments mapped onto the reference sequence. In one example, there are four short fragments from one read and positioned to different entries of the reference library, denoted as Y1, Y2, Y3, and Y4, each having a length of L1, L2, L3, and L4, respectively. The positioning positions are shown schematically in FIG. 3, where X1 and X2 are mapped to the reference sequence with overlapping positions, the length of the overlapping positions is J, and the connectivity length is (L1+ L2+ L3+ L4- J). In one example, the different short fragments are all *L* in length, and the predetermined threshold used herein is *L,* so that the alignment speed can be increased while allowing for the loss of data that is partially valid but of relatively low quality.

According to a specific embodiment of the present invention, after the second positioning result is obtained, S 130 further includes: according to a positioning result of short fragments from the (same) read in the second positioning result, a positioning result of the read is determined, and the read removed when its determination result does not meet a predetermined requirement. The read is removed and the short fragments corresponding to the read may also be removed. Therefore, on the premise of satisfying a certain sensitivity and specificity, an accurate mapping/local quick alignment can be directly carried out on the basis of the second positioning result, which can accelerate the alignment.

The evaluation method is not limited in this embodiment, and for example, a quantitative scoring method may be used. In one example, the positioning results of short fragments from the (same) read are scored, with the scoring rule being: the sites matching the reference sequence are added a negative number, and the sites not matching the reference sequence are added a positive number. After the second positioning result is obtained, according to the positioning result of the short fragments from the (same) read in the second positioning result, the positioning result of the read is scored, and the read removed when having a score no greater than a first preset value. According to a specific example, the read length is 25 bp, and sequence construction is performed on the short fragments from the (same) read to obtain a reconstructed sequence. For example, the base type of a certain site can be determined according to the support of more short sequences, if a certain site has no short fragment supported, namely no short fragment is aligned to the site, the base type of the site is uncertain and can be represented by N, so that a reconstructed sequence is obtained. It can be seen that the reconstructed sequence corresponds to the read, and the length of the reconstructed sequence is the read length. The site where the reconstructed sequence matches the reference sequence (ref) is subtracted by one, and the site where the reconstructed sequence does not match the reference sequence is added by one. The alignment error tolerance rate, namely the allowed mismatch ratio for one read/reconstructed sequence is 0.12, the length of the alignment allowable error is 3 bp (25 × 0.12), an initial score *Scoreᵢₙᵢₜ* is the read length, and the first preset value is 22 (25-3). In this way, reconstructed sequences whose score is less than 22, that is, the proportion of sites that do not match the reference sequence exceeds the alignment error tolerance rate are removed, which facilitates speeding up the alignment while allowing for the loss of data that is partially valid but of relatively low quality. According to a specific example, using bit arithmetic and dynamic programming algorithms [G. Myers. A fast bit-vector algorithm for approximate string matching based on dynamic progamming. Journal of the ACM, 46(3): 395-415, 1999], for each reconstructed sequence, the position of each site *i* is read in, a fast matching scoring is performed using a 64-bit binary mask, one point for each site, an initial Score *Scoreᵢₙᵢₜ* is the read length, which can be expressed as *Scoreᵢₙᵢₜ* = *length*(*read*), and the score *Score* is obtained by the matching scoring, which can be expressed as $Score = \left\{\begin{matrix}- 1 & read \left[i\right] = ref \left[j\right] , j ⊂ \left(i-1,i+1\right) \\ + 1 & read \left[i\right] ! = ref \left[j\right] , j ⊂ \left(i-1,i+1\right)\end{matrix}\right. .$

In one example, the positioning results of short fragments from the (same) read are scored, with the scoring rule being: the sites matching the reference sequence are added a positive number, and the sites not matching the reference sequence are added a negative number. After the second positioning result is obtained, according to the positioning result of the short fragments from the (same) read in the second positioning result, the positioning result of the read is scored, and short fragments corresponding to the read removed when having a score no less than a second preset value. According to a specific example, the read length is 25 bp, and sequence construction is performed on the short fragments from the (same) read to obtain a reconstructed sequence. For example, the base type of a certain site can be determined according to the support of more short sequences, if a certain site has no short fragment supported, namely no short fragment is aligned to the site, the base type of the site is uncertain and can be represented by N, so that a reconstructed sequence is obtained. It can be seen that the reconstructed sequence corresponds to the read, and the length of the reconstructed sequence is the read length. The site where the reconstructed sequence matches the reference sequence (ref) is added by one, and the site where the reconstructed sequence does not match the reference sequence is subtracted by one. The alignment error tolerance rate, namely the allowed mismatch ratio for one read/reconstructed sequence is 0.12, the length of the alignment allowable error is 3 bp (25 × 0.12), an initial score *Scoreᵢₙᵢₜ* is -25, and the second preset value is -22 (-25-3). In this way, reconstructed sequences whose score is greater than -22 are removed, which facilitates speeding up the alignment while allowing for the loss of data that is partially valid but of relatively low quality.

According to a specific embodiment of the present invention, the extending based on short fragments from the same read in the second positioning result at S 140 includes: a sequence construction is performed based on the short fragments from the same read to obtain a reconstructed sequence, and the extending is performed based on a common part of the reconstructed sequence and the reference sequence corresponding to the reconstructed sequence to obtain an extension sequence. In this way, the short fragment and the positioning information of the short fragment are converted into the positioning information of the read corresponding to the short fragment (referred to as the reconstructed sequence herein), which facilitates the subsequent alignment processing to be performed quickly and accurately.

The common part used herein may be the part common to multiple sequences. According to a specific embodiment of the present invention, the common part is a common substring and/or a common subsequence. The common substring may refer to a contiguous part that may be common to the plurality of sequences, and the common subsequence may need not be contiguous. For example, for ABCBDAB and BDCABA, the common subsequence is BCBA and the common substring is AB.

In one example, the performing the sequence construction based on the short fragments from the same read to obtain the reconstructed sequence used herein can determine the base type of a certain site on the reconstructed sequence according to the support of more short fragments, and if the short fragments which are not supported by a certain position. If a certain site has no short fragment supported, namely no short fragment is aligned to the site of the reference sequence, the base type of the site is uncertain and can be represented by N, so that the reconstructed sequence is obtained. It can be seen that the reconstructed sequence corresponds to the read, and the length of the reconstructed sequence is the read length.

The reference sequence corresponding to the reconstructed sequence used herein may be a reference sequence matched with the reconstructed sequence, and the length of the reference sequence may be no less than the read length. In one example, the length of the reference sequence corresponding to the reconstructed sequence is the same as that of the reconstructed sequence, and both are read length. In another example, the reconstructed sequence is allowed to be matched with the corresponding reference sequence in an error-tolerant manner, the length of the reference sequence corresponding to the reconstructed sequence is the length of the reconstructed sequence plus two times of the error-tolerant matching length. For example, the length of the reconstructed sequence, i.e., the read length, is 25 bp, the matching of the reconstructed sequence and the reference sequence is allowed to be mismatched by 12%, and the reference sequence aligned to the reconstructed sequence and the sequence of 3 bp (25 × 12%) at both ends of the reference sequence can be used as the reference sequence corresponding to the reconstructed sequence.

According to a specific embodiment of the present invention, the common part is a common substring, wherein optionally the extending based on short fragments from the same read in the second positioning result at S 140 includes: a common substring of the reconstructed sequence and the reference sequence corresponding to the reconstructed sequence is found, and a longest common substring of the reconstructed sequence and the reference sequence corresponding to the reconstructed sequence is determined; and the longest common substring is extended based on an edit distance to obtain an extension sequence. In this way, alignment results containing longer mapping sequences can be obtained more accurately.

According to a specific embodiment of the present invention, the common part is a common subsequence, wherein optionally the extending based on short fragments from the same read in the second positioning result at S 140 includes: a common subsequence of the reconstructed sequence and the reference sequence corresponding to the reconstructed sequence is found, and a longest common subsequence of the reconstructed sequence and the reference sequence corresponding to the reconstructed sequence is determined; and the longest common subsequence is extended based on an edit distance to obtain an extension sequence.

The edit distance used herein, also known as Levenshtein distance, may refer to the minimum number of editing operations required to change from one to another between two strings. Editing operations may include replacing a character with another, inserting a character, and deleting a character. Generally, the smaller the edit distance, the greater the similarity between the two strings.

In one example, for a reconstructed sequence/read, finding the longest common substring of the reconstructed sequence and the reference sequence corresponding to the reconstructed sequence can be expressed as solving the common substring of the two strings *x*₁*x*₂...*xᵢ* and *y*₁*y*₂...*yⱼ*, the length of which is m and n, respectively; and calculating the length of the common substring of the two strings to obtain a transfer equation: $c \left[i, j\right] = \left\{\begin{matrix}0 & i = 0 or j = 0 \\ c \left[i-1,j-1\right] + 1 & {x}_{i} = {y}_{j} \\ 0 & {x}_{i} \neq {y}_{j}\end{matrix}\right.$. The equation is solved to obtain the length of the longest common substring of the two sequences as max(*c*[*i*, *j*]), *i* ∈ {1,...,*m*}, *j* ∈ {1,...,*n*}. Then, the longest common substring is converted into the corresponding reference sequence by using the edit distance, so that both ends of the longest common substring can continuously grow, and the minimum character operations (replacement, deletion, and insertion) required between the two strings are found. The edit distance can be determined using a dynamic programming algorithm, the problem has an optimal substructure. The calculation of the edit distance *d*[*i*, *j*] can be expressed as the following formula: $d \left[i, j\right] = \left\{\begin{matrix}0 & i = 0 or j = 0 \\ min \left(d\left[i-1,j\right]+gap, d\left[i,j-1\right]+gap,d\left[i-1,j-1\right]+match\right) & {x}_{i} = {y}_{j} \\ min \left(d\left[i-1,j\right]+gap, d\left[i,j-1\right]+gap,d\left[i-1,j-1\right]+mismatch\right) & {x}_{i} \neq {y}_{j}\end{matrix}\right.$. Hole/Gap indicates the insertion or deletion of a character, and the gap in the formula indicates the penalty point required for inserting or deleting a character (corresponding to a site in the sequence). Match indicates that two characters are the same, and the match in the formula indicates a score when two characters are the same. Mismatch indicates that two characters are not equal/different, and the mismatch in the formula indicates a score when two characters are not equal/different. *d*[*i*, *j*] takes the smallest of the three. In a specific example, a gap penalty of 3 points, a consecutive gap increase penalty of 1 point, a site mismatch penalty of 2 points, and a site match score of 0. In this way, it is advantageous to the efficient alignment of gap-containing sequences.

According to a specific embodiment of the present invention, the common part is a common subsequence. According to a specific embodiment of the present invention, S140 includes: a common subsequence of the short fragments positioned to the same entry of the reference library in the second positioning result is found, and a longest common subsequence corresponding to each read is determined; and the longest common subsequence is extended based on an edit distance to obtain an extension sequence.

In one example, for a reconstructed sequence/read, the longest common subsequence of the reconstructed sequence and the reference sequence corresponding to the reconstructed sequence is found. Based on the longest common subsequence, the reconstructed sequence corresponding to the longest common subsequence is converted into the reference sequence corresponding to the longest common subsequence. The edit distance of the two sequences can be found out by utilizing Smith Waterman algorithm. For the two strings *x*₁*x*₂...*xᵢ* and *y*₁*y*₂...*yᵢ*, it can be obtained by the following formula: $F \left[i, j\right] = max \left\{\begin{matrix}0 i = 0 or j = 0 \\ F \left(i-1,j-1\right) + σ \left(i, j\right) \\ F \left(i-1,j\right) + σ \left(-,j\right) \\ F \left(i,j-1\right) + σ \left(i,-\right)\end{matrix}\right.$. *σ* represents a scoring function, *σ*(*i*, *j*) represents the score of the mismatch or match of the characters (sites) x*ᵢ* and y*ⱼ*, *σ*(-, *j*) represents the score for vacancy (deletion) of x*ᵢ* or insertion of y*ⱼ*, and *σ*(*i,* -) represents the score for deletion of y*ⱼ* or insertion of x*ᵢ*. Then, the method of calculating the edit distance in the previous example can be used to convert the segment of the reconstructed sequence corresponding to the longest common subsequence into the reference sequence corresponding to the reconstructed sequence, and the segment of the reconstructed sequence corresponding to the longest common subsequence can be continuously grown at both ends to find out the minimum character operation (replacement, deletion, insertion). In a specific example, a gap penalty of 3 points, a consecutive gap increase penalty of 1 point, a site mismatch penalty of 2 points, and a site match score of 4 points. In this way, efficient alignment of gap-containing sequences can be achieved and sequences containing both gaps and other sites with high accuracy can be retained.

According to a specific embodiment of the present invention, S 140 further includes: the extension sequence is truncated from at least one end of the extension sequence, a proportion of error positioning sites of a truncated extension sequence is calculated, and the truncation is stopped when the following condition is satisfied: the proportion of the error positioning sites of the truncated extension sequence is less than a third preset value. In this way, by using the truncation and culling methods, the local sequence with good matching can be well reserved, which is conducive to improving the efficiency of the data.

Specifically, according to a specific embodiment of the present invention, the extension sequence is truncated based on the following: i. a first error rate and a second error rate are calculated; if the first error rate is less than the second error rate, the extension sequence is truncated from a first end of the extension sequence, and if the first error rate is greater than the second error rate, the extension sequence is truncated from a second end of the extension sequence to obtain a truncated extension sequence, wherein the first error rate used herein is a proportion of error positioning sites of the truncated extension sequence obtained by truncating the extension sequence from the first end of the extension sequence, and the second error rate used herein is a proportion of error positioning sites of the truncated extension sequence obtained by truncating the extension sequence from the second end of the extension sequence, ii) i) is performed by replacing the extension sequence with the truncated extension sequence until the proportion of the error positioning sites of the truncated extension sequence is less than a fourth preset value. In this way, by using the double-end truncation and culling methods, the local sequence with good matching can be well reserved, which is conducive to improving the efficiency of the data. According to a specific example, the length of the extension sequence is 25 bp, and the fourth preset value and the third preset value are 0.12.

According to a specific embodiment of the present invention, S140 further includes: a sliding window is performed on the extension sequence from at least one end of the extension sequence, and a proportion of error positioning sites of a window sequence obtained by the sliding window is calculated; and the extension sequence is truncated according to the proportion of the error positioning sites of the window sequence, and the truncation is stopped when the following condition is satisfied: the proportion of the error positioning sites of the window sequence obtained by the sliding window is greater than a fifth preset value. In this way, by using the truncation and culling methods, the local sequence with good matching can be well reserved, which is conducive to improving the efficiency of the data.

Specifically, according to a specific embodiment of the present invention, the extension sequence is truncated based on the following: i. a third error rate and a fourth error rate are calculated; if the third error rate is less than the fourth error rate, the extension sequence is truncated from a second end of the extension sequence, and if the third error rate is greater than the fourth error rate, the extension sequence is truncated from a first end of the extension sequence to obtain a truncated extension sequence, wherein the third error rate used herein is a proportion of error positioning sites of a window sequence obtained by performing window sliding on the extension sequence from the first end of the extension sequence, and the fourth error rate used herein is a proportion of error positioning sites of a window sequence obtained by performing window sliding on the extension sequence from the second end of the extension sequence, ii) i) is performed by replacing the extension sequence with the truncated extension sequence until the proportion of the error positioning sites of the truncated extension sequence is less than a sixth preset value. In this way, by using the double-end truncation and culling methods, the local sequence with good matching can be well reserved, which is conducive to improving the efficiency of the data.

According to a specific embodiment of the present invention, the window of the sliding window is no greater than the length of the extension sequence. According to a specific example, the length of the extension sequence is 25 bp, the window size of the sliding window is 10 bp, and the sixth preset value and the fifth preset value are 0.12.

According to a specific embodiment of the present invention, the truncation has a size of 1 bp, that is, one truncation is to remove one base. In this way, the alignment results containing more long sequences can be obtained efficiently.

In a specific example, sequence alignment is performed on the same batch of simulated data using Bowtie (http://bowtie-bio.sourceforge.net/index.shtml), BWA (http://bio-bwa.sourceforge.net/), and the aforementioned alignment method, in which the simulated data is based on the human reference genome setting and contains 100K sequences with a length of 100 bp. The space, time, proportion of reference sequences on the alignment (Map rate), and accuracy required for each software/method run are comparable. In this example, the time and memory required by using the alignment method in this embodiment is slightly longer and greater than those of Bowtie or BWA, but the alignment accuracy and the proportion on sequence alignment by using the alignment method in this embodiment is 98.9% and 99.9%, both slightly higher than those using Bowtie and BWA.

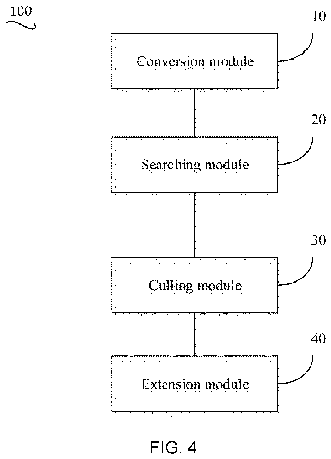

The device of claim 8 is preferably configured to implement the method of claim 1, optionally in any one of the aforementioned embodiments/implementations. Accordingly, referring to FIG. 4, an alignment device according to an embodiment of the present invention is provided, wherein the device 100 includes a conversion module 10 configured to convert, in case of more than one read, each read into a set of short fragments of length *L* corresponding to the respective read to obtain a plurality of sets of short fragments, a searching module 20 configured to determine a corresponding position of the short fragments in a reference library to obtain a first positioning result, wherein the reference library is a hash table constructed based on a reference sequence, the reference library includes a plurality of entries, each entry of the reference library corresponds to a seed sequence of length *L*, and the seed sequence is configured to match at least one sequence on the reference sequence, wherein a distance on the reference sequence between two seed sequences that correspond to two adjacent entries of the reference library is less than the length of the short fragments, a culling module 30 configured to remove short fragments positioned on any one of the adjacent entries of the reference library in the first positioning result to obtain a second positioning result, and an extension module 40 configured to extend based on short fragments from the same read in the second positioning result to obtain an alignment result of the read.

The foregoing description of the technical features and effects of the alignment method in any one of the embodiments is also applicable to the alignment device in this embodiment of the present invention, and details are not described herein again.

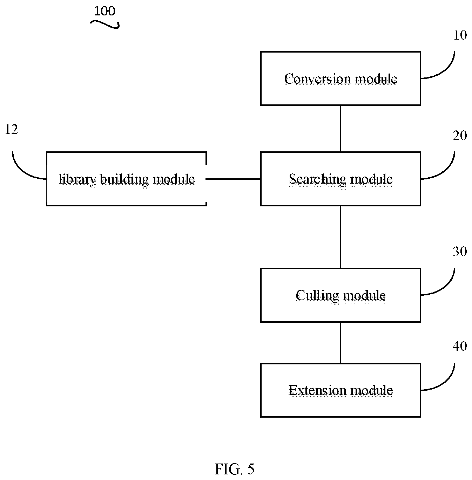

For example, according to a specific embodiment of the present invention, referring to FIG. 5, the device 100 further includes a library building module 12 configured to build the reference library and to perform the following by using the library building module 12: a length *L* of the seed sequence is determined according to total number of bases *totalBase* of the reference sequence, *L* = *µ* * *log*(*totalBase*), *µ* ⊂ [1,3]; all possible seed sequences are generated based on the length of the seed sequence to obtain a seed sequence set; and a seed sequence capable of matching the reference sequence in the seed sequence set and a matching position of the seed sequence are determined to obtain the reference library.

According to a specific embodiment of the present invention, the determining the seed sequence capable of matching the reference sequence in the seed sequence set and the matching position of the seed sequence includes: a sliding window is performed on the reference sequence by using a window having a size of *L,* the seed sequence in the seed sequence set is matched with a window sequence obtained by the sliding window to determine the seed sequence capable of matching the reference sequence in the seed sequence set and the matching position of the seed, and an error tolerance rate for the matching is *ε*₁.

According to a specific embodiment of the present invention, a step size of performing the sliding window is determined in accordance with L and *ε*₁.

According to a specific embodiment of the present invention, a step size of performing the sliding window is no less than *L* * *ε*₁.

According to a specific embodiment of the invention , a distance between two adjacent entries of the reference library is greater than a step size of the sliding window.

According to a specific embodiment of the present invention, the conversion module 10 is configured to perform a sliding window on the read by using a window having a size of *L* to obtain a set of short fragments corresponding to the read, and a step size of performing the sliding window is 1 bp.

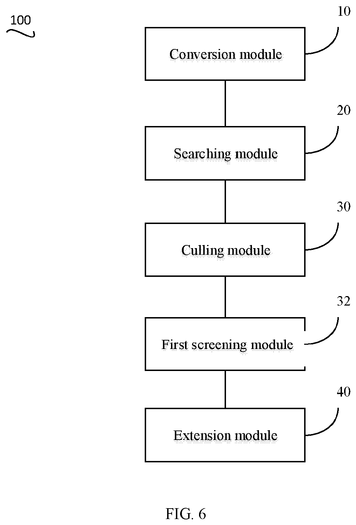

According to a specific embodiment of the present invention, referring to FIG. 6, a first screening module 32 is further included: the first screening module 32 is coupled to the culling module 30 and configured to perform the following on the second positioning result from the culling module 30: removing short fragments having a connectivity length less than a predetermined threshold, and replacing the second positioning result with a removed result, wherein the connectivity length is a total length of short fragments in the second positioning result (which is herein also referred to as second alignment result) that are from the (same) read and are positioned to different entries of the reference library mapped to the reference sequence.

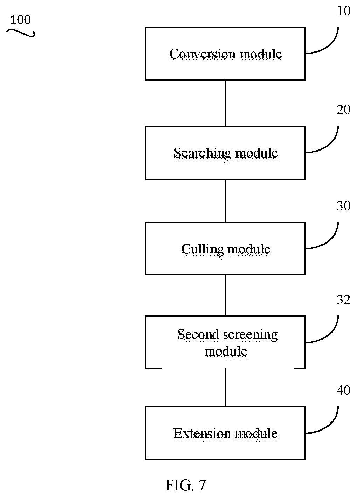

According to a specific embodiment of the present invention, referring to FIG. 7, a second screening module 34 is further included: the second screening module 34 is coupled to the removal module 30 and configured to determine a positioning result of the read according to a positioning result of short fragments from the (same) read in the second positioning result, and remove short fragments corresponding to the read whose determination result does not meet a predetermined requirement.

According to a specific embodiment of the present invention, the second screening module 34 is configured to score the positioning result of the read according to the positioning result of the short fragments from the (same) read in the second positioning result, and remove the read when having a score no greater than a first preset value.

According to a specific embodiment of the present invention, the second screening module 34 is configured to score the positioning result of the read according to the positioning result of the short fragments from the (same) read in the second positioning result, and remove the read when having a score no less than a second preset value.

According to a specific embodiment of the present invention, the extension module 40 is configured to perform a sequence construction based on the short fragments from the same read to obtain a reconstructed sequence; and extend based on a common part of the reconstructed sequence and the reference sequence corresponding to the reconstructed sequence to obtain an extension sequence.

According to a specific embodiment of the present invention, the common part is a common substring. The extension module 40 may be configured to find a common substring of the reconstructed sequence and the reference sequence corresponding to the reconstructed sequence, and determine a longest common substring of the reconstructed sequence and the reference sequence corresponding to the reconstructed sequence; and extend the longest common substring based on an edit distance to obtain an extension sequence.

According to a specific embodiment of the present invention, the common part is a common subsequence. The extension module 40 may be configured to find a common subsequence of the reconstructed sequence and the reference sequence corresponding to the reconstructed sequence, and determine a longest common subsequence of the reconstructed sequence and the reference sequence corresponding to the reconstructed sequence; and extend the longest common subsequence based on an edit distance to obtain an extension sequence.

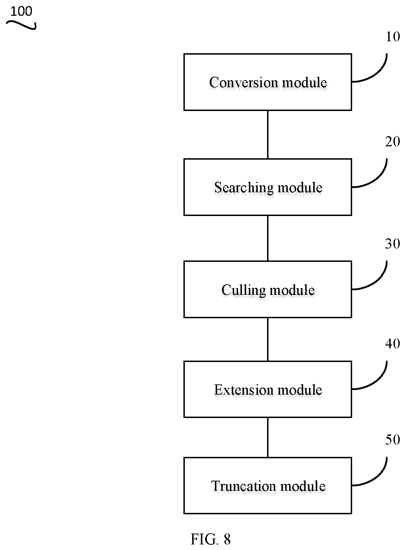

According to a specific embodiment of the present invention, referring to FIG. 8, a truncation module 50 is further provided, which is configured to truncate the extension sequence from at least one end of the extension sequence from the extension module 40, calculate a proportion of error positioning sites of a truncated extension sequence, and stop the truncation when the following condition is satisfied: the proportion of the error positioning sites of the truncated extension sequence is less than a third preset value.

According to the specific embodiment of the present invention, a truncation module 50 is further included and is configured to: i) calculate a first error rate and a second error rate; if the first error rate is less than the second error rate, truncate the extension sequence from a first end of the extension sequence to obtain a truncated extension sequence, and if the first error rate is greater than the second error rate, truncate the extension sequence from a second end of the extension sequence to obtain a truncated extension sequence, wherein the first error rate is a proportion of error positioning sites of the truncated extension sequence obtained by truncating the extension sequence from the first end of the extension sequence, and the second error rate is a proportion of error positioning sites of the truncated extension sequence obtained by truncating the extension sequence from the second end of the extension sequence; and ii) perform i) by replacing the extension sequence with the truncated extension sequence until the proportion of the error positioning sites of the truncated extension sequence is less than a fourth preset value.

According to the specific embodiment of the present invention, a truncation module 50 is further included and is configured to perform a sliding window on the extension sequence from at least one end of the extension sequence, and calculate a proportion of error positioning sites of a window sequence obtained by the sliding window, and truncate the extension sequence according to the proportion of the error positioning sites of the window sequence, and stop the truncation when the following condition is satisfied: the proportion of the error positioning sites of the window sequence obtained by the sliding window is greater than a fifth preset value.

According to the specific embodiment of the present invention, a truncation module 50 is further included and is configured to: i) calculate a third error rate and a fourth error rate; if the third error rate is less than the fourth error rate, truncate the extension sequence from a second end of the extension sequence to obtain a truncated extension sequence, and if the third error rate is greater than the fourth error rate, truncate the extension sequence from a first end of the extension sequence to obtain a truncated extension sequence, wherein the third error rate is a proportion of error positioning sites of a window sequence obtained by performing window sliding on the extension sequence from the first end of the extension sequence, and the fourth error rate is a proportion of error positioning sites of a window sequence obtained by performing window sliding on the extension sequence from the second end of the extension sequence; and ii) perform i) by replacing the extension sequence with the truncated extension sequence until the proportion of the error positioning sites of the window sequence is greater than the sixth preset value.

According to a specific embodiment of the present invention, the truncation has a size of 1 bp.

According to a specific embodiment of the present invention, the window of the sliding window is no greater than a length of the extension sequence.

A computer readable medium is provided according to the present invention, the medium is configured to carry a part or all of the steps of the alignment method in any one of the foregoing embodiments. The medium used herein may include, but may not be limited to, a read-only memory, a random access memory, a magnetic disk or an optical disk, and the like.

The present invention further provides an alignment system, and the system 1000 includes an input device 100 configured to input data; an output device 200 configured to output data; a processor 300 configured to execute a computer executable program, and the executing the computer executable program includes performing the alignment method of the present invention including any one of the foregoing embodiments. The system further includes a storage device 400 configured to store data. The storage device 400 includes the computer executable program.

Herein, references to the description of the term "one embodiment", "some embodiments", "an example", "a specific example", or "some examples", etc., mean that a particular feature, structure, material, or characteristic described in connection with the embodiment or example is included in at least one embodiment or example. In this specification, a schematic representation of the foregoing terms does not necessarily refer to the same embodiments or examples. Furthermore, the particular features, structures, materials, or characteristics described may be combined in any suitable manner in any one or more embodiments or examples.

## Claims

1. A computer-implemented alignment method, comprising:
converting each read into a set of short fragments of length *L*, to obtain a plurality of sets of short fragments; determining a corresponding position of the short fragments in a reference library by aligning the short fragments with the reference library to obtain a first positioning result,
wherein the reference library is a hash table constructed based on a reference sequence, the reference library comprises a plurality of entries, each entry of the reference library corresponds to a seed sequence of length *L*, and the seed sequence is configured to match at least one sequence on the reference sequence,
wherein a distance on the reference sequence between two seed sequences that correspond to two adjacent entries of the reference library is less than the length of the short fragments;
removing short fragments positioned on any one of the adjacent entries of the reference library in the first positioning result to obtain a second positioning result; and
extending based on short fragments from the same read in the second positioning result to obtain an alignment result of the read.

2. A method as claimed in claim 1, wherein constructing the reference library comprises:
determining a length *L* of the seed sequence according to total number of bases *totalBase* of the reference sequence, *L* = *µ * log*(*totalBase*), *µ* ⊂ [1, 3] ;
generating all possible seed sequences based on a length of the seed sequence to obtain a seed sequence set; and
determining a seed sequence capable of matching the reference sequence in the seed sequence set and a matching position of the seed sequence, optionally comprising:
performing a sliding window on the reference sequence by using a window having a size of *L*, matching the seed sequence in the seed sequence set with a window sequence obtained by the sliding window to determine the seed sequence capable of matching the reference sequence in the seed sequence set and the matching position of the seed sequence, wherein an error tolerance rate for the matching is *ε*₁, to obtain the reference library;
wherein optionally, a step size of performing the sliding window is determined in accordance with *L* and *ε*₁;
wherein optionally a step size of performing the sliding window is no less than *L***ε*₁;
wherein further optionally a distance between two adjacent entries of the reference library is greater than or equal to a step size of the sliding window.

3. A method as claimed in claim 1 or 2, wherein converting each read into the set of short fragments comprises:
performing a sliding window on the read by using a window having a size of *L* to obtain a set of short fragments corresponding to the read, and a step size of performing the sliding window is 1 bp; and/or
wherein determining the corresponding position of the short fragments in the reference library to obtain the first positioning result comprises:
matching the short fragments with seed sequences corresponding to entries of the reference library to determine the positions of the short fragments in the reference library, wherein an error tolerance rate for the matching is *ε*₂; and/or
after obtaining the second positioning result, the method comprising:
removing short fragments having a connectivity length less than a predetermined threshold, and replacing the second positioning result with a removed result, wherein the connectivity length is a total length of short fragments in the second positioning result that are from the read and are positioned to different entries of the reference library mapped to the reference sequence;
optionally determining a positioning result of the read according to a positioning result of short fragments from the read in the second positioning result, and removing the read when its determination result does not meet a predetermined requirement; further optionally comprising:
scoring the positioning result of the read according to the positioning result of the short fragments from the read in the second positioning result, and removing the read when having a score no greater than a first preset value, or
scoring the positioning result of the read according to the positioning result of the short fragments from the read in the second positioning result, and removing the read when having a score no less than a second preset value.

4. A method as claimed in any of claims 1 to 3, wherein the extending based on short fragments from the read in the second positioning result comprises:
performing a sequence construction based on the short fragments from the read to obtain a reconstructed sequence; and
extending based on a common part of the reconstructed sequence and the reference sequence corresponding to the reconstructed sequence to obtain an extension sequence.

5. A method as claimed in claim 4, wherein the common part is a common substring; wherein optionally the extending based on short fragments from the read in the second positioning result comprises:
finding a common substring of the reconstructed sequence and the reference sequence corresponding to the reconstructed sequence, and determining a longest common substring of the reconstructed sequence and the reference sequence corresponding to the reconstructed sequence; and
extending the longest common substring based on an edit distance to obtain an extension sequence.

6. A method as claimed in any of claim 4, wherein the common part is a common subsequence;
wherein optionally the extending based on short fragments from the read in the second positioning result comprises:
finding a common subsequence of the reconstructed sequence and the reference sequence corresponding to the reconstructed sequence, and determining a longest common subsequence of the reconstructed sequence and the reference sequence corresponding to the reconstructed sequence; and
extending the longest common subsequence based on an edit distance to obtain an extension sequence.

7. A method as claimed in claim 5 or 6, further comprising:
truncating the extension sequence from at least one end of the extension sequence, calculating a proportion of error positioning sites of a truncated extension sequence, and stopping the truncation when the following condition is satisfied: the proportion of the error positioning sites of the truncated extension sequence is less than a third preset value; or
the method further comprising:
i) calculating a first error rate and a second error rate, if the first error rate is less than the second error rate, truncating the extension sequence from a first end of the extension sequence,
if the first error rate is greater than the second error rate, truncating the extension sequence from a second end of the extension sequence to obtain a truncated extension sequence,
wherein the first error rate is a proportion of error positioning sites of the truncated extension sequence obtained by truncating the extension sequence from the first end of the extension sequence,
the second error rate is a proportion of error positioning sites of the truncated extension sequence obtained by truncating the extension sequence from the second end of the extension sequence;
ii) performing i) by replacing the extension sequence with the truncated extension sequence until the proportion of the error positioning sites of the truncated extension sequence is less than a fourth preset value; or
the method further comprising:
performing a sliding window on the extension sequence from at least one end of the extension sequence, and calculating a proportion of error positioning sites of a window sequence obtained by the sliding window, truncating the extension sequence according to the proportion of the error positioning sites of the window sequence, and stopping the truncation when the following condition is satisfied: the proportion of the error positioning sites of the window sequence obtained by the sliding window is greater than a fifth preset value, wherein optionally, the window of the sliding window is no greater than a length of the extension sequence;
or
the method further comprising:
i) calculating a third error rate and a fourth error rate, if the third error rate is less than the fourth error rate, truncating the extension sequence from a second end of the extension sequence,
if the third error rate is greater than the fourth error rate, truncating the extension sequence from a first end of the extension sequence to obtain a truncated extension sequence,
wherein the third error rate is a proportion of error positioning sites of a window sequence obtained by performing window sliding on the extension sequence from the first end of the extension sequence,
the fourth error rate is a proportion of error positioning sites of a window sequence obtained by performing window sliding on the extension sequence from the second end of the extension sequence;
ii) performing i) by replacing the extension sequence with the truncated extension sequence until the proportion of the error positioning sites of the window sequence is greater than the sixth preset value, wherein optionally, the window of the sliding window is no greater than a length of the extension sequence;
and
wherein further optionally the truncation has a size of 1 bp.

8. An alignment device, comprising:
a conversion module configured to convert each read into a set of short fragments of length *L* corresponding to the read, to obtain a plurality of sets of short fragments;
a searching module configured to determine a corresponding position of the short fragments in a reference library to obtain a first positioning result,
wherein the reference library is a hash table constructed based on a reference sequence, the reference library comprises a plurality of entries, each entry of the reference library corresponds to a seed sequence of length L, and the seed sequence is configured to match at least one sequence on the reference sequence,
wherein a distance on the reference sequence between two seed sequences that correspond to two adjacent entries of the reference library is less than the length of the short fragments;
a culling module configured to remove short fragments positioned on any one of the adjacent entries of the reference library in the first positioning result to obtain a second positioning result; and
an extension module configured to extend based on short fragments from the same read in the second positioning result to obtain an alignment result of the read.

9. A device as claimed in claim 8, further comprising a library building module configured to construct the reference library and to perform:
determining a length *L* of the seed sequence according to total number of bases *totalBase* of the reference sequence, *L* = *µ ** log(*totalBase*), *µ* ⊂ [1, 3] ;
generating all possible seed sequences based on a length of the seed sequence to obtain a seed sequence set; and
determining a seed sequence capable of matching the reference sequence in the seed sequence set and a matching position of the seed sequence to obtain the reference library, optionally comprising:
performing a sliding window on the reference sequence by using a window having a size of *L*, matching the seed sequence in the seed sequence set with a window sequence obtained by the sliding window to determine the seed sequence capable of matching the reference sequence in the seed sequence set and the matching position of the seed sequence, wherein an error tolerance rate for the matching is *ε*₁;
wherein optionally a step size of performing the sliding window is determined in accordance with *L* and *ε*₁; or
wherein optionally a step size of performing the sliding window is no less than *L * ε*₁; or
wherein optionally a distance between two adjacent entries of the reference library is greater than a step size of the sliding window;
wherein further optionally the conversion module is configured to:
perform a sliding window on the read by using a window having a size of *L* to obtain a set of short fragments corresponding to the read, and a step size of performing the sliding window is 1 bp;
and/or the device further comprising a first screening module, coupled to the culling module and configured to perform the following on the second positioning result from the culling module:
removing short fragments having a connectivity length less than a predetermined threshold, and replacing the second positioning result with a removed result, wherein the connectivity length is a total length of short fragments in the second positioning result that are from the read and are positioned to different entries of the reference library mapped to the reference sequence;
and/or the device further comprising a second screening module coupled to the removal module and configured to:
determine, according to a positioning result of short fragments from the read in the second positioning result, a positioning result of the read, and remove short fragments corresponding to the read whose determination result does not meet a predetermined requirement,
wherein optionally the second screening module is configured to:
score, according to the positioning result of the short fragments from the read in the second positioning result, the positioning result of the read, and remove the read when having a score no greater than a first preset value; or
score, according to the positioning result of the short fragments from the read in the second positioning result, the positioning result of the read, and remove the read when having a score no less than a second preset value;
and/or wherein the extension module is configured to:
perform a sequence construction based on the short fragments from the read to obtain a reconstructed sequence; and
extend based on a common part of the reconstructed sequence and the reference sequence corresponding to the reconstructed sequence to obtain an extension sequence.

10. A device as claimed in claim 9, wherein the common part is a common substring;
wherein optionally the extension module is configured to:
find a common substring of the reconstructed sequence and the reference sequence corresponding to the reconstructed sequence, and determine a longest common substring of the reconstructed sequence and the reference sequence corresponding to the reconstructed sequence; and
extend the longest common substring based on an edit distance to obtain an extension sequence;
the device optionally further comprising:
a truncation module configured to:
truncate the extension sequence from at least one end of the extension sequence from the extension module, calculate a proportion of error positioning sites of a truncated extension sequence, and stop the truncation when the following condition is satisfied: the proportion of the error positioning sites of the truncated extension sequence is less than a third preset value;
the truncation module being optionally further configured to:
i) calculate a first error rate and a second error rate, if the first error rate is less than the second error rate, truncate the extension sequence from a first end of the extension sequence to obtain a truncated extension sequence,
if the first error rate is greater than the second error rate, truncate the extension sequence from a second end of the extension sequence to obtain a truncated extension sequence,
wherein the first error rate is a proportion of error positioning sites of the truncated extension sequence obtained by truncating the extension sequence from the first end of the extension sequence,
the second error rate is a proportion of error positioning sites of the truncated extension sequence obtained by truncating the extension sequence from the second end of the extension sequence;
ii) perform i) by replacing the extension sequence with the truncated extension sequence until the proportion of the error positioning sites of the truncated extension sequence is less than a fourth preset value;
the truncation module being optionally further configured to:
perform a sliding window on the extension sequence from at least one end of the extension sequence, and calculate a proportion of error positioning sites of a window sequence obtained by the sliding window,
truncate the extension sequence according to the proportion of the error positioning sites of the window sequence, and stop the truncation when the following condition is satisfied: the proportion of the error positioning sites of the window sequence obtained by the sliding window is greater than a fifth preset value, optionally, the window of the sliding window is no greater than a length of the extension sequence; and
the truncation module being optionally further configured to:
i) calculate a third error rate and a fourth error rate, if the third error rate is less than the fourth error rate, truncate the extension sequence from a second end of the extension sequence to obtain a truncated extension sequence,
if the third error rate is greater than the fourth error rate, truncate the extension sequence from a first end of the extension sequence to obtain a truncated extension sequence,
wherein the third error rate is a proportion of error positioning sites of a window sequence obtained by performing window sliding on the extension sequence from the first end of the extension sequence,
the fourth error rate is a proportion of error positioning sites of a window sequence obtained by performing window sliding on the extension sequence from the second end of the extension sequence;
ii) perform i) by replacing the extension sequence with the truncated extension sequence until the proportion of the error positioning sites of the window sequence is greater than the sixth preset value; optionally, the window of the sliding window is no greater than a length of the extension sequence.

11. A device as claimed in claim 9, wherein the common part is a common subsequence;
wherein the extension module is optionally configured to:
find a common subsequence of the reconstructed sequence and the reference sequence corresponding to the reconstructed sequence, and determine a longest common subsequence of the reconstructed sequence and the reference sequence corresponding to the reconstructed sequence; and
extend the longest common subsequence based on an edit distance to obtain an extension sequence;
the truncation module being optionally configured to:
truncate the extension sequence from at least one end of the extension sequence from the extension module, calculate a proportion of error positioning sites of a truncated extension sequence, and stop the truncation when the following condition is satisfied: the proportion of the error positioning sites of the truncated extension sequence is less than a third preset value;
the truncation module being optionally further configured to:
i) calculate a first error rate and a second error rate, if the first error rate is less than the second error rate, truncate the extension sequence from a first end of the extension sequence to obtain a truncated extension sequence,
if the first error rate is greater than the second error rate, truncate the extension sequence from a second end of the extension sequence to obtain a truncated extension sequence,
wherein the first error rate is a proportion of error positioning sites of the truncated extension sequence obtained by truncating the extension sequence from the first end of the extension sequence,
the second error rate is a proportion of error positioning sites of the truncated extension sequence obtained by truncating the extension sequence from the second end of the extension sequence;
ii) perform i) by replacing the extension sequence with the truncated extension sequence until the proportion of the error positioning sites of the truncated extension sequence is less than a fourth preset value;
further comprising a truncation module configured to:
perform a sliding window on the extension sequence from at least one end of the extension sequence, and calculate a proportion of error positioning sites of a window sequence obtained by the sliding window,
truncate the extension sequence according to the proportion of the error positioning sites of the window sequence, and stop the truncation when the following condition is satisfied: the proportion of the error positioning sites of the window sequence obtained by the sliding window is greater than a fifth preset value, wherein optionally, the window of the sliding window is no greater than a length of the extension sequence;
the truncation module being optionally further configured to:
i) calculate a third error rate and a fourth error rate, if the third error rate is less than the fourth error rate, truncate the extension sequence from a second end of the extension sequence to obtain a truncated extension sequence,
if the third error rate is greater than the fourth error rate, truncate the extension sequence from a first end of the extension sequence to obtain a truncated extension sequence,
wherein the third error rate is a proportion of error positioning sites of a window sequence obtained by performing window sliding on the extension sequence from the first end of the extension sequence,
the fourth error rate is a proportion of error positioning sites of a window sequence obtained by performing window sliding on the extension sequence from the second end of the extension sequence;
ii) perform i) by replacing the extension sequence with the truncated extension sequence until the proportion of the error positioning sites of the window sequence is greater than the sixth preset value, wherein further optionally, the window of the sliding window is no greater than a length of the extension sequence.

12. A device as claimed in any of claims 9 to 11, wherein the truncation has a size of 1 bp.

13. An alignment system, comprising:
an input device configured to input data;
an output device configured to output data;
a processor configured to execute a computer executable program, the executing the computer executable program comprising performing the method of any one of claims 1 to 7; and
a storage device configured to store data, wherein the storage device comprises the computer executable program.

14. A computer readable medium comprising instructions which, when executed by a computer, cause the computer to carry out the method of any one of claims 1 to 7.

## Patentansprüche

1. Eine computerimplementiertes Alignment-Verfahren, aufweisend:
Konvertieren jedes Reads in eine Menge kurzer Fragmente der Länge L, um eine Vielzahl von Mengen kurzer Fragmente zu erhalten;
Bestimmen einer entsprechenden Position der kurzen Fragmente in einer Referenzbibliothek durch Abgleichen der kurzen Fragmente mit der Referenzbibliothek, um ein Ergebnis der ersten Positionierung zu erhalten,
wobei die Referenzbibliothek eine Hashtabelle ist, die auf einer Referenzsequenz basiert, die Referenzbibliothek eine Vielzahl von Einträgen aufweist; jeder Eintrag der Referenzbibliothek einer Seed-Sequenz der Länge L̅ entspricht, und die Seed-Sequenz so konfiguriert ist, dass sie mit mindestens einer Sequenz auf der Referenzsequenz übereinstimmt,
wobei ein Abstand auf der Referenzsequenz zwischen zwei Seed-Sequenzen, die zwei benachbarten Einträgen der Referenzbibliothek entsprechen, kleiner ist als die Länge der kurzen Fragmente;
Entfernen kurzer Fragmente, die auf einem der benachbarten Einträge der Referenzbibliothek im ersten Positionierungsergebnis positioniert sind, um ein zweites Positionierungsergebnis zu erhalten; und
Verlängern basierend auf kurzen Fragmenten aus demselben Read im zweiten Positionierungsergebnis, um ein Alignment-Ergebnis des Reads zu erhalten.

2. Verfahren, wie in Anspruch 1 beansprucht, wobei Konstruieren der Referenzbibliothek aufweist:
Bestimmen einer Länge *L* der Seed-Sequenz entsprechend der Gesamtzahl der Basen *totalBase* der Referenzsequenz, *L* = *µ* log*(*totalBase*), *µ* ⊂ [1, 3];
Erzeugen aller möglichen Seed-Sequenzen basierend auf einer Länge der Seed-Sequenz, um eine Seed-Sequenzmenge zu erhalten; und
Bestimmen einer Seed-Sequenz, die in der Lage ist mit der Referenzsequenz in der Seed-Sequenzmenge übereinzustimmen, und einer passenden Position der Seed-Sequenz, optional aufweisend:
Durchführen eines gleitenden Fensters auf der Referenzsequenz durch Verwendung eines Fensters mit der Größe von *L*, Abgleichen der Seed-Sequenz in der Seed-Sequenzmenge mit einer Fenstersequenz, die durch das gleitende Fenster erhalten wurde, um die Seed-Sequenz, die in der Lage ist mit der Referenzsequenz in der Seed-Sequenzmenge übereinzustimmen, und die übereinstimmende Position der Seed-Sequenz zu bestimmen, wobei eine Fehlertoleranzrate für das Abgleichen *ε*₁ ist, um die Referenzbibliothek zu erhalten;
wobei optional eine Schrittgröße für das Ausführen des gleitenden Fensters gemäß *L* und *ε*₁ bestimmt wird;
wobei optional eine Schrittweite für das Ausführen des gleitenden Fensters nicht kleiner als ist *L***ε*₁*;*
wobei ferner optional ein Abstand zwischen zwei benachbarten Einträgen der Referenzbibliothek größer oder gleich einer Schrittweite des gleitenden Fensters ist.

3. Verfahren, wie in Anspruch 1 oder 2 beansprucht, wobei Konvertieren jedes Reads in die Menge kurzer Fragmente aufweist:
Durchführen eines gleitenden Fensters auf dem Read unter Verwendung eines Fensters mit einer Größe *L,* um eine Menge kurzer Fragmente zu erhalten, die dem Read entspricht, und eine Schrittweite für das Ausführen des gleitenden Fensters beträgt 1 bp; und/oder
wobei Bestimmen der entsprechenden Position der kurzen Fragmente in der Referenzbibliothek, um das erste Positionierungsergebnis zu erhalten, aufweist:
Abgleichen der kurzen Fragmente mit Seed-Sequenzen, die den Einträgen der Referenzbibliothek entsprechen, um die Positionen der kurzen Fragmente in der Referenzbibliothek zu bestimmen, wobei eine Fehlertoleranz für das Matching beträgt *ε*₂; und/oder
nach Erhalt des zweiten Positionierungsergebnisses weist das Verfahren auf:
Entfernen kurzer Fragmente mit einer Konnektivitätslänge kleiner als eine vorgegebene Schwelle und Ersetzen des zweiten Positionierungsergebnisses durch ein entferntes Ergebnis, wobei die Konnektivitätslänge die Gesamtlänge der kurzen Fragmente im zweiten Positionierungsergebnis ist, die aus dem Read stammen und auf verschiedenen Einträge der Referenzbibliothek positioniert sind, die auf die Referenzsequenz abgebildet ist;
optional Bestimmen eines Positionierungsergebnisses des Reads entsprechend einem Positionierungsergebnis kurzer Fragmente aus dem Read im zweiten Positionierungsergebnis, und Entfernen des Reads, wenn sein Bestimmungsergebnis eine vorgegebene Anforderung nicht erfüllt; ferner optional enthaltend:
Bewerten des Positionierungsergebnisses des Reads entsprechend dem Positionierungsergebnis der kurzen Fragmente aus dem Read im zweiten Positionierungsergebnis, und Entfernen des Reads, wenn er eine Wertung nicht größer als ein erster voreingestellter Wert aufweist, oder
Bewerten des Positionierungsergebnisses des Reads entsprechend dem Positionierungsergebnis der kurzen Fragmente aus dem Read im zweiten Positionierungsergebnis, und Entfernen des Reads, wenn er eine Wertung nicht kleiner als ein zweiter voreingestellter Wert aufweist.

4. Verfahren, wie in einem der Ansprüche 1 bis 3 beansprucht, bei dem Verlängern auf Basis kurzer Fragmente aus dem Read in dem zweiten Positionierungsergebnis aufweist:
Durchführen einer Sequenzkonstruktion auf Basis der kurzen Fragmente aus dem Read, um eine rekonstruierte Sequenz zu erhalten; und
Verlängern basierend auf einem gemeinsamen Teil der rekonstruierten Sequenz und der Referenzsequenz, die der rekonstruierten Sequenz entspricht, um eine Verlängerungssequenz zu erhalten.

5. Verfahren, wie in Anspruch 4 beansprucht, bei dem der gemeinsame Teil eine gemeinsame Subzeichenkette ist;
wobei optional das Verlängern basierend auf kurzen Fragmenten aus dem Read in dem zweiten Positionierungsergebnis aufweist:
Finden einer gemeinsamen Subzeichenkette der rekonstruierten Sequenz und der Referenzsequenz, die der rekonstruierten Sequenz entspricht, und Bestimmen einer längsten gemeinsamen Subzeichenkette der rekonstruierten Sequenz und der Referenzsequenz, die der rekonstruierten Sequenz entspricht;
Verlängern der längsten gemeinsamen Subzeichenkette basierend auf einer Bearbeitungsdistanz, um eine Verlängerungssequenz zu erhalten.

6. Verfahren, wie in Anspruch 4 beansprucht, wobei der gemeinsame Teil eine gemeinsame Subsequenz ist; wobei optional das Verlängern basierend auf kurzen Fragmenten aus dem Read in dem zweiten Positionierungsergebnis aufweist:
Finden einer gemeinsamen Subsequenz der rekonstruierten Sequenz und der Referenzsequenz, die der rekonstruierten Sequenz entspricht, und Bestimmen einer längsten gemeinsamen Subsequenz der rekonstruierten Sequenz und der Referenzsequenz, die der rekonstruierten Sequenz entspricht; und
Verlängern der längsten gemeinsamen Subsequenz basierend auf einer Bearbeitungsdistanz, um eine Verlängerungssequenz zu erhalten.

7. Verfahren, wie in Anspruch 5 oder 6 beansprucht, ferner aufweisend:
Trunkieren der Verlängerungssequenz von mindestens einem Ende der Verlängerungssequenz, Berechnen eines Anteils der Fehlerpositionierungsstellen einer trunkierten Verlängerungssequenz und Stoppen der Trunkierung, wenn folgende Bedingung erfüllt ist: der Anteil der Fehlerpositionierungsstellen der trunkierten Verlängerungssequenz ist kleiner als ein dritter voreingestellter Wert; oder
das Verfahren ferner aufweisend:
i) Berechnen einer ersten und einer zweiten Fehlerrate, falls die erste Fehlerrate geringer ist als die zweite Fehlerrate, Trunktieren der Verlängerungssequenz vom ersten Ende der Verlängerungssequenz,
falls die erste Fehlerrate größer als die zweite Fehlerrate ist, Trunkieren der Verlängerungssequenz von einem zweiten Ende der Verlängerungssequenz, um eine trunkierte Verlängerungssequenz zu erhalten,
wobei die erste Fehlerrate ein Anteil der Fehlerpositionierungsstellen der trunkierten Verlängerungssequenz ist, die durch Trunkierung der Verlängerungssequenz vom ersten Ende der Erweiterungssequenz erhalten wurde,
die zweite Fehlerrate ein Anteil der Fehlerpositionierungsstellen der trunkierten Verlängerungssequenz ist, die durch Trunkieren der Verlängerungssequenz vom zweiten Ende der Verlängerungssequenz erhalten wurde;
ii) Durchführen von i) durch Ersetzen der Verlängerungssequenz mit der trunkierten Verlängerungssequenz, bis der Anteil der Fehlerpositionierungsstellen der trunkierten Verlängerungssequenz kleiner als ein vierter voreingestellter Wert ist; oder
die Methode ferner aufweisend:
Durchführen eines gleitenden Fensters auf der Verlängerungssequenz von mindestens einem Ende der Verlängerungssequenz und Berechnen eines Anteils der Fehlerpositionierungsstellen einer Fenstersequenz, die durch das gleitende Fenster erhalten wurde,
Trunkieren der Verlängerungssequenz entsprechend dem Anteil der Fehlerpositionierungsstellen der Fenstersequenz und Stoppen der Trunkierung, wenn folgende Bedingung erfüllt ist: der Anteil der Fehlerpositionierungsstellen der Fenstersequenz, die durch das gleitende Fenster erhalten wurde, ist größer als ein fünfter voreingestellter Wert, wobei optional das Fenster des gleitenden Fensters nicht größer als eine Länge der Verlängerungssequenz ist; oder
das Verfahren ferner aufweisend:
i) Berechnen einer dritten Fehlerrate und einer vierten Fehlerrate, falls die dritte Fehlerrate kleiner als die vierte Fehlerrate ist, Trunkieren der Verlängerungssequenz von einem zweiten Ende der Verlängerungssequenz,
falls die dritte Fehlerrate größer als die vierte Fehlerrate ist, Trunkieren der Verlängerungssequenz von einem ersten Ende der Erweiterungssequenz, um eine c zu erhalten,
wobei die dritte Fehlerrate ein Anteil der Fehlerpositionierungsstellen einer Fenstersequenz ist, die durch Ausführen von Fenstergleiten auf der Verlängerungssequenz vom ersten Ende der Verlängerungssequenz erhalten wurde,
die vierte Fehlerrate ist ein Anteil der Fehlerpositionierungsstellen einer Fenstersequenz, die durch Ausführen von Fenstergleiten auf der Verlängerungssequenz vom zweiten Ende der Verlängerungssequenz erhalten wurde;
ii) Durchführen von i) durch Ersetzen der Verlängerungssequenz mit der trunkierten Verlängerungssequenz, bis der Anteil der Fehlerpositionierungsstellen der Fenstersequenz größer ist als der sechste voreingestellte Wert, wobei optional das Fenster des gleitenden Fensters nicht größer als eine Länge der Verlängerungssequenz ist; und
wobei ferner optional die Trunkierung eine Größe von 1 bp hat.

8. Ein Alignment-Gerät, aufweisend:
ein Konversionsmodul, das eingerichtet ist jeden Read in eine Menge kurzer Fragmente der Länge L umzuwandeln, die dem Read entspricht, um eine Vielzahl von Mengen kurzer Fragmente zu erhalten;
ein Suchmodul, das eingerichtet ist eine entsprechende Position der kurzen Fragmente in einer Referenzbibliothek zu bestimmen, um ein erstes Positionierungsergebnis zu erhalten,
wobei die Referenzbibliothek eine Hashtabelle ist, die auf Basis einer Referenzsequenz konstruiert ist, die Referenzbibliothek eine Vielzahl von Einträgen aufweist, jeder Eintrag der Referenzbibliothek einer Seed-Sequenz der Länge *L* entspricht, und die Seed-Sequenz eingerichtet ist mit mindestens einer Sequenz auf der Referenzsequenz übereinzustimmen,
wobei ein Abstand auf der Referenzsequenz zwischen zwei Seed-Sequenzen, die zwei benachbarten Einträgen der Referenzbibliothek entsprechen, kleiner ist als die Länge der kurzen Fragmente;
ein Auswahlmodul, das eingerichtet ist kurze Fragmente zu entfernen, die auf einem der benachbarten Einträge der Referenzbibliothek im ersten Positionierungsergebnis positioniert sind, um ein zweites Positionierungsergebnis zu erhalten; und
ein Verlängerungsmodul, das eingerichtet ist basierend auf kurzen Fragmenten desselben Reads im zweiten Positionierungsergebnis zu verlängern, um ein Alignment-Ergebnis des Reads zu erhalten.

9. Gerät, wie in Anspruch 8 beansprucht, das ferner ein Bibliotheksbaumodul aufweist, das zur Erstellung der Referenzbibliothek und zur Ausführung des Folgenden eingerichtet ist:
Bestimmen einer Länge *L* der Seed-Sequenz entsprechend der Gesamtzahl der Basen *totalBase* der Referenzsequenz, *L* = *µ* log*(*totalBase*), *µ* ⊂ [1, 3];
Erzeugen aller möglichen Seed-Sequenzen basierend auf einer Länge der Seed-Sequenz, um eine Seed-Sequenzmenge zu erhalten; und
Bestimmen einer Seed-Sequenz, die in der Lage ist mit der Referenzsequenz im Seed-Sequenzmenge übereinzustimmen, und einer übereinstimmenden Position der Seed-Sequenz, um die Referenzbibliothek zu erhalten, optional aufweisend:
Durchführen eines gleitenden Fensters auf der Referenzsequenz durch Verwendung eines Fensters mit einer Größe von *L*, Abgleichen der Seed-Sequenz in der Seed-Sequenzmenge mit einer Fenstersequenz, die durch das gleitende Fenster erhalten wird, um die Seed-Sequenz, die in der Lage ist mit der Referenzsequenz in der Seed-Sequenzmenge übereinzustimmen, und die übereinstimmende Position der Seed-Sequenz zu bestimmen, wobei eine Fehlertoleranzrate für das Abgleichen *ε*₁ ist;
wobei optional eine Schrittlänge für das Ausführen des gleitenden Fensters gemäß *L* und *ε*₁ bestimmt wird; oder
wobei optional eine Schrittweite für das Ausführen des gleitenden Fensters nicht kleiner als *L***ε*₁ ist; oder
wobei optional der Abstand zwischen zwei benachbarten Einträgen der Referenzbibliothek größer ist als die Schrittweite des gleitenden Fensters;
wobei ferner optional das Umwandlungsmodul eingerichtet ist:
ein gleitendes Fenster auf dem Read auszuführen, indem ein Fenster mit der Größe L verwendet wird, um eine Menge kurzer Fragmente zu erhalten, die dem Read entsprechen, und eine Schrittlänge für das gleitende Fenster beträgt 1 bp;
und/oder das Gerät weist ferner ein erstes Überprüfungsmodul auf, das mit dem Auswahlmodul gekoppelt und eingerichtet ist das Folgende beim zweiten Positionierungsergebnis des Auswahlmoduls auszuführen:
Entfernen kurzer Fragmente mit einer Konnektivitätslänge kleiner als eine vorgegebene Schwelle und Ersetzen des zweiten Positionierungsergebnisses durch ein entferntes Ergebnis, wobei die Konnektivitätslänge die Gesamtlänge der kurzen Fragmente im zweiten Positionierungsergebnis ist, die aus dem Read stammen und auf verschiedene Einträge der Referenzbibliothek positioniert sind, die auf die Referenzsequenz abgebildet sind;
und/oder das Gerät weist ferner ein zweites Überprüfungsmodul auf, das mit dem Auswahlmodul gekoppelt und eingerichtet ist:
ein Positionierungsergebnis des Reads zu bestimmen gemäß einem Positionierungsergebnis der kurzen Fragmente aus dem Read im zweiten Positionierungsergebnis und kurze Fragmente zu entfernen, die dem Read entsprechen, dessen Bestimmung eine vorgegebene Anforderung nicht erfüllt,
wobei optional das zweite Überprüfungsmodul eingerichtet ist:
das Positionierungsergebnis des Reads entsprechend dem Positionierungsergebnis der kurzen Fragmente aus dem Read im zweiten Positionierungsergebnis zu bewerten und den Read zu entfernen, wenn er eine Bewertung aufweist, die nicht größer als ein erster voreingestellter Wert ist, oder
das Positionierungsergebnisses des Reads entsprechend dem Positionierungsergebnis der kurzen Fragmente aus dem Read im zweiten Positionierungsergebnis zu bewerten, und den Read zu entfernen, wenn er eine Wertung aufweist, die nicht kleiner als ein zweiter voreingestellter Wert ist.
und/oder wobei das Verlängerungsmodul eingerichtet ist:
eine Sequenzkonstruktion auf Basis der kurzen Fragmente aus dem Read auszuführen, um eine rekonstruierte Sequenz zu erhalten; und
basierend auf einem gemeinsamen Teil der rekonstruierten Sequenz und der Referenzsequenz, die der rekonstruierten Sequenz entspricht, zu verlängern, um eine Erweiterungssequenz zu erhalten.

10. Gerät, wie in Anspruch 9 beansprucht, wobei der gemeinsame Teil eine gemeinsame Subzeichenkette ist;
wobei optional das Erweiterungsmodul eingerichtet ist:
eine gemeinsame Subzeichenkette der rekonstruierten Sequenz und der Referenzsequenz zu finden, die der rekonstruierten Sequenz entspricht, und eine längste gemeinsame Subzeichenkette der rekonstruierten Sequenz sowie der Referenzsequenz zu finden, die der rekonstruierten Sequenz entspricht; und
die längste gemeinsame Subzeichenkette basierend auf einer Bearbeitungsdistanz zu verlängern, um eine Verlängerungssequenz zu erhalten;
wobei das Gerät ferner optional aufweist:
ein Trunkierungsmodul, eingerichtet zum:
Trunkieren der Verlängerungssequenz von mindestens einem Ende der Verlängerungssequenz von dem Verlängerungsmodul, Berechnen eines Anteils der Fehlerpositionierungsstellen einer trunkierten Verlängerungssequenz und Stoppen der Trunkierung, wenn folgende Bedingung erfüllt ist: der Anteil der Fehlerpositionierungsstellen der trunkierten Verlängerungssequenz ist kleiner als ein dritter voreingestellter Wert;
wobei das Trunkierungsmodul ferner optional eingerichtet ist zum:
i) Berechnen einer ersten und einer zweiten Fehlerrate, falls die erste Fehlerrate geringer ist als die zweite Fehlerrate, Trunktieren der Verlängerungssequenz vom ersten Ende der Verlängerungssequenz, um eine trunkierte Verlängerungssequenz zu erhalten,
falls die erste Fehlerrate größer als die zweite Fehlerrate ist, Trunkieren der Verlängerungssequenz von einem zweiten Ende der Verlängerungssequenz, um eine trunkierte Verlängerungssequenz zu erhalten,
wobei die erste Fehlerrate ein Anteil der Fehlerpositionierungsstellen der trunkierten Verlängerungssequenz ist, die durch Trunkierung der Verlängerungssequenz vom ersten Ende der Erweiterungssequenz erhalten wurde,
die zweite Fehlerrate ein Anteil der Fehlerpositionierungsstellen der trunkierten Verlängerungssequenz ist, die durch Trunkieren der Verlängerungssequenz vom zweiten Ende der Verlängerungssequenz erhalten wurde;
ii) Durchführen von i) durch Ersetzen der Verlängerungssequenz mit der trunkierten Verlängerungssequenz, bis der Anteil der Fehlerpositionierungsstellen der trunkierten Verlängerungssequenz kleiner als ein vierter voreingestellter Wert ist; oder
wobei das Trunkierungsmodul ferner optional eingerichtet ist zum:
Durchführen eines gleitenden Fensters auf der Verlängerungssequenz von mindestens einem Ende der Verlängerungssequenz und Berechnen eines Anteils der Fehlerpositionierungsstellen einer Fenstersequenz, die durch das gleitende Fenster erhalten wurde,
Trunkieren der Verlängerungssequenz entsprechend dem Anteil der Fehlerpositionierungsstellen der Fenstersequenz und Stoppen der Trunkierung, wenn folgende Bedingung erfüllt ist: der Anteil der Fehlerpositionierungsstellen der Fenstersequenz, die durch das gleitende Fenster erhalten wurde, ist größer als ein fünfter voreingestellter Wert, wobei optional das Fenster des gleitenden Fensters nicht größer als eine Länge der Verlängerungssequenz ist; und
wobei das Trunkierungsmodul ferner optional eingerichtet ist zum:
i) Berechnen einer dritten Fehlerrate und einer vierten Fehlerrate, falls die dritte Fehlerrate kleiner als die vierte ist, Trunkieren der Verlängerungssequenz von einem zweiten Ende der Verlängerungssequenz,
falls die dritte Fehlerrate größer als die vierte Fehlerrate ist, Trunkieren der Verlängerungssequenz von einem ersten Ende der Erweiterungssequenz, um eine trunkierte Erweiterungssequenz zu erhalten,
wobei die dritte Fehlerrate ein Anteil der Fehlerpositionierungsstellen einer Fenstersequenz ist, die durch Ausführen von Fenstergleiten auf der Verlängerungssequenz vom ersten Ende der Verlängerungssequenz erhalten wurde,
die vierte Fehlerrate ist ein Anteil der Fehlerpositionierungsstellen einer Fenstersequenz, die durch Ausführen von Fenstergleiten auf der Verlängerungssequenz vom zweiten Ende der Verlängerungssequenz erhalten wurde;
ii) Durchführen von i) durch Ersetzen der Verlängerungssequenz mit der trunkierten Verlängerungssequenz, bis der Anteil der Fehlerpositionierungsstellen der Fenstersequenz größer ist als der sechste voreingestellte Wert; wobei optional das Fenster des gleitenden Fensters nicht größer als eine Länge der Verlängerungssequenz ist.

11. Gerät, wie in Anspruch 9 beansprucht, wobei der gemeinsame Teil eine gemeinsame Subsequenz ist; wobei das Verlängerungsmodul optional eingerichtet ist zum:
Finden einer gemeinsamen Subsequenz der rekonstruierten Sequenz und der Referenzsequenz, die der rekonstruierten Sequenz entspricht, und Bestimmen einer längsten gemeinsamen Subsequenz der rekonstruierten Sequenz sowie der Referenzsequenz, die der rekonstruierten Sequenz entspricht; und
Verlängern der längsten gemeinsamen Subsequenz basierend auf einer Bearbeitungsdistanz, um eine Verlängerungssequenz zu erhalten;
wobei das Trunkierungsmodul optional eingerichtet ist zum:
Trunkieren der Verlängerungssequenz von mindestens einem Ende der Verlängerungssequenz vom Verlängerungsmodul, Berechnen eines Anteils der Fehlerpositionierungsstellen einer trunkierten Verlängerungssequenz und Stoppen der Trunkierung, wenn folgende Bedingung erfüllt ist: der Anteil der Fehlerpositionierungsstellen der trunkierten Verlängerungssequenz ist kleiner als ein dritter voreingestellter Wert;
wobei das Trunkierungsmodul ferner optional eingerichtet ist zum:
i) Berechnen einer ersten und einer zweiten Fehlerrate, falls die erste Fehlerrate geringer ist als die zweite Fehlerrate, Trunktieren der Verlängerungssequenz vom ersten Ende der Verlängerungssequenz, um eine trunkierte Verlängerungssequenz zu erhalten,
falls die erste Fehlerrate größer als die zweite Fehlerrate ist, Trunkieren der Verlängerungssequenz von einem zweiten Ende der Verlängerungssequenz, um eine trunkierte Verlängerungssequenz zu erhalten,
wobei die erste Fehlerrate ein Anteil der Fehlerpositionierungsstellen der trunkierten Verlängerungssequenz ist, die durch Trunkierung der Verlängerungssequenz vom ersten Ende der Erweiterungssequenz erhalten wurde,
die zweite Fehlerrate ein Anteil der Fehlerpositionierungsstellen der trunkierten Verlängerungssequenz ist, die durch Trunkieren der Verlängerungssequenz vom zweiten Ende der Verlängerungssequenz erhalten wurde;
ii) Durchführen von i) durch Ersetzen der Verlängerungssequenz mit der trunkierten Verlängerungssequenz, bis der Anteil der Fehlerpositionierungsstellen der trunkierten Verlängerungssequenz kleiner als ein vierter voreingestellter Wert ist;
ferner ein Trunkierungsmodul aufweisend, eingerichtet zum:
Durchführen eines gleitenden Fensters auf der Verlängerungssequenz von mindestens einem Ende der Verlängerungssequenz und Berechnen eines Anteils der Fehlerpositionierungsstellen einer Fenstersequenz, die durch das gleitende Fenster erhalten wurde,
Trunkieren der Verlängerungssequenz entsprechend dem Anteil der Fehlerpositionierungsstellen der Fenstersequenz und Stoppen der Trunkierung, wenn folgende Bedingung erfüllt ist: der Anteil der Fehlerpositionierungsstellen der Fenstersequenz, die durch das gleitende Fenster erhalten wurde, ist größer als ein fünfter voreingestellter Wert, wobei optional das Fenster des gleitenden Fensters nicht größer als eine Länge der Verlängerungssequenz ist; und
wobei das Trunkierungsmodul ferner optional eingerichtet ist zum:
i) Berechnen einer dritten Fehlerrate und einer vierten Fehlerrate, falls die dritte Fehlerrate kleiner als die vierte ist, Trunkieren der Verlängerungssequenz von einem zweiten Ende der Verlängerungssequenz,
falls die dritte Fehlerrate größer als die vierte Fehlerrate ist, Trunkieren der Verlängerungssequenz von einem ersten Ende der Erweiterungssequenz, um eine trunkierte Erweiterungssequenz zu erhalten,
wobei die dritte Fehlerrate ein Anteil der Fehlerpositionierungsstellen einer Fenstersequenz ist, die durch Ausführen von Fenstergleiten auf der Verlängerungssequenz vom ersten Ende der Verlängerungssequenz erhalten wurde,
die vierte Fehlerrate ist ein Anteil der Fehlerpositionierungsstellen einer Fenstersequenz, die durch Ausführen von Fenstergleiten auf der Verlängerungssequenz vom zweiten Ende der Verlängerungssequenz erhalten wurde;
ii) Durchführen von i) durch Ersetzen der Verlängerungssequenz mit der trunkierten Verlängerungssequenz, bis der Anteil der Fehlerpositionierungsstellen der Fenstersequenz größer ist als der sechste voreingestellte Wert; wobei ferner optional das Fenster des gleitenden Fensters nicht größer als eine Länge der Verlängerungssequenz ist.

12. Gerät, wie in einem der Ansprüche 9 bis 11 beansprucht, bei dem die Trunkation eine Größe von 1 bp hat.

13. Ein Alignment-System, aufweisend:
ein Eingabegerät, das eingerichtet ist Daten einzugeben;
ein Ausgabegerät, das eingerichtet ist Daten auszugeben;
ein Prozessor, der eingerichtet ist ein computerausführbares Programm auszuführen, wobei das Ausführen des ausführbaren Programms Ausführe des Verfahrens eines der Ansprüche 1 bis 7 aufweiset; und
ein Speichergerät, das eingerichtet ist zum Speichern von Daten, wobei das Speichergerät das computerausführbare Programm aufweist.

14. Ein computerlesbares Medium, das Anweisungen aufweist, die, wenn sie von einem Computer ausgeführt werden, den Computer dazu bringen, das Verfahren eins der Ansprüche 1 bis 7 auszuführen.

## Revendications

1. Méthode d'alignement mis en œuvre par ordinateur, comprenant:
convertir de chaque lecture en un ensemble de fragments courts de longueur L, afin d'obtenir une pluralité d'ensembles de fragments courts ;
la détermination d'une position correspondante des fragments courts dans une bibliothèque de référence en alignant les fragments courts avec la bibliothèque de référence afin d'obtenir un premier résultat de positionnement,
dans lequel la bibliothèque de référence est une table de hachage construite à partir d'une séquence de référence, la bibliothèque de référence comprend une pluralité d'entrées, chaque entrée de la bibliothèque de référence correspond à une séquence de départ de longueur *L*, et la séquence de départ est configurée pour correspondre à au moins une séquence de la séquence de référence,
dans lequel une distance, sur la séquence de référence, entre deux séquences de départ correspondant à deux entrées adjacentes de la bibliothèque de référence est inférieure à la longueur des fragments courts ;
supprimer les fragments courts positionnés sur l'une quelconque des entrées adjacentes de la bibliothèque de référence dans le premier résultat de positionnement afin d'obtenir un deuxième résultat de positionnement; et
étendre, sur la base des fragments courts issus de la même lecture, le deuxième résultat de positionnement afin d'obtenir un résultat d'alignement de la lecture.

2. Méthode selon la revendication 1, dans lequel la construction de la bibliothèque de référence comprend:
déterminer d'une longueur L de la séquence de départ en fonction du nombre total de bases *totalBase* de la séquence de référence, *L* = *µ**log(*totalBase*), *µ* ⊂ [1, 3] ;
générer de toutes les séquences de départ possibles sur la base de cette longueur de la séquence de départ afin d'obtenir un ensemble de séquences de départ ; et
déterminer, au sein de l'ensemble de séquences de départ, d'une séquence de départ capable de s'aligner sur la séquence de référence ainsi que d'une position d'alignement de cette séquence de départ, comprenant éventuellement:
appliquer une fenêtre glissante à la séquence de référence à l'aide d'une fenêtre de taille *L*, la mise en correspondance de la séquence de départ de l'ensemble de séquences de départ avec une séquence de fenêtre obtenue par la fenêtre glissante afin de déterminer la séquence de départ capable de correspondre à la séquence de référence dans l'ensemble de séquences de départ et la position de correspondance de cette séquence de départ, le taux de tolérance d'erreur pour la mise en correspondance étant *ε*₁, afin d'obtenir la bibliothèque de référence;
dans lequel, de manière facultative, une taille de pas pour l'exécution de la fenêtre glissante est déterminée en fonction de *L* et de *ε*₁;
dans lequel, de manière facultative, une taille de pas pour l'exécution de la fenêtre glissante n'est pas inférieure à *L* * *ε*₁ ;
dans lequel, de manière encore facultative, une distance entre deux entrées adjacentes de la bibliothèque de référence est supérieure ou égale à une taille de pas de la fenêtre glissante.

3. Méthode selon la revendication 1 ou 2, dans lequel convertir de chaque séquence lue en un ensemble de fragments courts comprend:
appliquer une fenêtre glissante à la séquence lue à l'aide d'une fenêtre de taille *L* afin d'obtenir un ensemble de fragments courts correspondant à la séquence lue, le pas de la fenêtre glissante étant de 1 pb; et/ou dans lequel déterminer de la position correspondante des fragments courts dans la bibliothèque de référence afin d'obtenir le premier résultat de positionnement comprend:
miser en correspondance des fragments courts avec des séquences d'amorçage correspondant à des entrées de la bibliothèque de référence afin de déterminer les positions des fragments courts dans la bibliothèque de référence, le taux de tolérance d'erreur pour cette mise en correspondance étant *ε*₂ ; et/ou après obtenir du deuxième résultat de positionnement, la méthode comprenant:
éliminer des fragments courts ayant une longueur de connectivité inférieure à un seuil prédéterminé, et remplacer du deuxième résultat de positionnement par un résultat éliminé, la longueur de connectivité étant la longueur totale des fragments courts du deuxième résultat de positionnement qui proviennent de la lecture et sont positionnés sur différentes entrées de la bibliothèque de référence mappées à la séquence de référence;
déterminer, de manière facultative, un résultat de positionnement du séquençat en fonction d'un résultat de positionnement de fragments courts issus du séquençat dans le deuxième résultat de positionnement, et supprimer le séquençat lorsque son résultat de détermination ne satisfait pas à une exigence prédéterminée;
comprenant en outre, de manière facultative:
attribuer d'un score au résultat de positionnement de la lecture en fonction du résultat de positionnement des fragments courts issus de la lecture dans le deuxième résultat de positionnement, et éliminer la lecture lorsque son score n'est pas supérieur à une première valeur prédéfinie, ou
attribuer d'un score au résultat de positionnement de la lecture en fonction du résultat de positionnement des fragments courts issus de la lecture dans le deuxième résultat de positionnement, et éliminer la lecture lorsque son score n'est pas inférieur à une deuxième valeur prédéfinie.

4. Méthode selon l'une quelconque des revendications 1 à 3, dans lequel étendre, basée sur des fragments courts issus de la lecture du deuxième résultat de positionnement, comprend:
réaliser d'une construction de séquence à partir des fragments courts issus de la lecture afin d'obtenir une séquence reconstruite; et
étendre basée sur une partie commune de la séquence reconstruite et de la séquence de référence correspondant à la séquence reconstruite afin d'obtenir une séquence d'extension.

5. Méthode selon la revendication 4, dans lequel la partie commune est une sous-chaîne commune; dans lequel, de manière facultative, l'extension à partir de fragments courts issus de la lecture dans le deuxième résultat de positionnement comprend:
trouver une sous-chaîne commune de la séquence reconstruite et de la séquence de référence correspondant à la séquence reconstruite, et la détermination d'une sous-chaîne commune la plus longue de la séquence reconstruite et de la séquence de référence correspondant à la séquence reconstruite; et
étendre la sous-chaîne commune la plus longue sur la base d'une distance d'édition afin d'obtenir une séquence d'extension.

6. Méthode selon l'une quelconque des revendications 4, dans lequel la partie commune est une sous-séquence commune; dans lequel, de manière facultative, l'extension sur la base de courts fragments issus de la lecture dans le deuxième résultat de positionnement comprend:
trouver une sous-séquence commune à la séquence reconstruite et à la séquence de référence correspondant à la séquence reconstruite, et la détermination d'une sous-séquence commune la plus longue entre la séquence reconstruite et la séquence de référence correspondant à la séquence reconstruite; et
étendre la sous-séquence commune la plus longue sur la base d'une distance d'édition afin d'obtenir une séquence d'extension.

7. Méthode selon la revendication 5 ou 6, comprenant en outre:
tronquer la séquence d'extension à partir d'au moins une extrémité de celle-ci, le calcul d'une proportion de sites de positionnement erronés d'une séquence d'extension tronquée, arrêter la troncature lorsque la condition suivante est satisfaite: la proportion de sites de positionnement erronés de la séquence d'extension tronquée est inférieure à une troisième valeur prédéfinie ; ou
la méthode comprenant en outre:
i) calculer un premier taux d'erreur et d'un deuxième taux d'erreur ; si le premier taux d'erreur est inférieur au deuxième taux d'erreur, tronquer la séquence d'extension à partir d'une première extrémité de celle-ci, si le premier taux d'erreur est supérieur au deuxième taux d'erreur, tronquer la séquence d'extension à partir d'une deuxième extrémité de celle-ci pour obtenir une séquence d'extension tronquée,
où le premier taux d'erreur est une proportion des sites de positionnement erronés de la séquence d'extension tronquée obtenue en tronquant la séquence d'extension à partir de la première extrémité de celle-ci,
le deuxième taux d'erreur est une proportion des sites de positionnement erronés de la séquence d'extension tronquée obtenue en tronquant la séquence d'extension à partir de la deuxième extrémité de celle-ci ;
ii) exécuter i) en remplaçant la séquence d'extension par la séquence d'extension tronquée jusqu'à ce que la proportion de sites de positionnement d'erreur de la séquence d'extension tronquée soit inférieure à une quatrième valeur prédéfinie; ou
la méthode comprenant en outre:
appliquer une fenêtre glissante à la séquence d'extension à partir d'au moins une extrémité de celle-ci, et calculer une proportion de sites de positionnement erronés d'une séquence de fenêtre obtenue par la fenêtre glissante,
tronquer la séquence d'extension en fonction de la proportion des sites de positionnement erronés de la séquence de fenêtre, et arrêter de la troncature lorsque la condition suivante est satisfaite: la proportion de sites de positionnement erronés de la séquence de fenêtre obtenue par la fenêtre glissante est supérieure à une cinquième valeur prédéfinie, dans laquelle, de manière facultative, la fenêtre de la fenêtre glissante n'est pas supérieure à une longueur de la séquence d'extension; ou
la méthode comprenant en outre :
i) calculer un troisième taux d'erreur et d'un quatrième taux d'erreur; si le troisième taux d'erreur est inférieur au quatrième taux d'erreur, tronquer de la séquence d'extension à partir d'une deuxième extrémité de celle-ci,
si le troisième taux d'erreur est supérieur au quatrième taux d'erreur, tronquer la séquence d'extension à partir d'une première extrémité de celle-ci afin d'obtenir une séquence d'extension tronquée,
le troisième taux d'erreur étant une proportion de sites de positionnement erronés d'une séquence de fenêtre obtenue en effectuant un glissement de fenêtre sur la séquence d'extension à partir de la première extrémité de celle-ci,
le quatrième taux d'erreur étant une proportion de sites de positionnement erronés d'une séquence de fenêtre obtenue en effectuant un glissement de fenêtre sur la séquence d'extension à partir de la deuxième extrémité de celle-ci;
ii) exécuter i) en remplaçant la séquence d'extension par la séquence d'extension tronquée jusqu'à ce que la proportion de sites de positionnement d'erreur de la séquence de fenêtre soit supérieure à la sixième valeur prédéfinie, dans lequel, de manière facultative, la fenêtre de la fenêtre coulissante n'est pas supérieure à la longueur de la séquence d'extension; et
dans lequel, de manière encore facultative, la troncature a une taille de 1 pb.

8. Dispositif d'alignement, comprenant:
un module de conversion configuré pour convertir chaque séquence lue en un ensemble de fragments courts de longueur L correspondant à ladite séquence lue, afin d'obtenir une pluralité d'ensembles de fragments courts;
un module de recherche configuré pour déterminer une position correspondante des fragments courts dans une bibliothèque de référence afin d'obtenir un premier résultat de positionnement,
dans lequel la bibliothèque de référence est une table de hachage construite à partir d'une séquence de référence, la bibliothèque de référence comprend une pluralité d'entrées, chaque entrée de la bibliothèque de référence correspond à une séquence de départ de longueur *L*, et la séquence de départ est configurée pour correspondre à au moins une séquence de la séquence de référence,
dans lequel une distance, sur la séquence de référence, entre deux séquences de départ correspondant à deux entrées adjacentes de la bibliothèque de référence est inférieure à la longueur des fragments courts;
un module de élimination configuré pour éliminer les fragments courts situés sur l'une quelconque des entrées adjacentes de la bibliothèque de référence dans le premier résultat de positionnement afin d'obtenir un deuxième résultat de positionnement; et
un module d'extension configuré pour effectuer une extension à partir des fragments courts issus de la même lecture dans le deuxième résultat de positionnement afin d'obtenir un résultat d'alignement de la lecture.

9. Dispositif selon la revendication 8, comprenant en outre un module de construction de bibliothèque configuré pour construire la bibliothèque de référence et pour effectuer les opérations suivantes:
déterminer une longueur *L* de la séquence de départ en fonction du nombre total de bases *totalBase* de la séquence de référence, *L* = *µ**log(*totalBase*), *µ* ⊂ [1, 3];
générer toutes les séquences de départ possibles en fonction de la longueur de la séquence de départ afin d'obtenir un ensemble de séquences de départ; et
déterminer, dans l'ensemble de séquences de départ, une séquence de départ capable de s'aligner sur la séquence de référence ainsi qu'une position d'alignement de ladite séquence de départ afin d'obtenir la bibliothèque de référence, comprenant en outre, de manière facultative:
appliquer une fenêtre glissante à la séquence de référence à l'aide d'une fenêtre de taille *L*, la mise en correspondance de la séquence d'amorçage de l'ensemble de séquences d'amorçage avec une séquence de fenêtre obtenue par la fenêtre glissante afin de déterminer la séquence d'amorçage capable de correspondre à la séquence de référence dans l'ensemble de séquences d'amorçage et la position de correspondance de la séquence d'amorçage, dans lequel un taux de tolérance d'erreur pour la mise en correspondance est *ε*₁;
dans lequel, de manière facultative, une taille de pas pour l'exécution de la fenêtre glissante est déterminée en fonction de L et de *ε*₁; ou
dans lequel, de manière facultative, une taille de pas pour l'exécution de la fenêtre glissante n'est pas inférieure à *L***ε*₁; ou
dans lequel, de manière facultative, une distance entre deux entrées adjacentes de la bibliothèque de référence est supérieure à une taille de pas de la fenêtre glissante;
dans lequel, en outre de manière facultative, le module de conversion est configuré pour:
appliquer une fenêtre glissante sur la séquence lue à l'aide d'une fenêtre de taille *L* afin d'obtenir un ensemble de fragments courts correspondant à la séquence lue, le pas de appliquer la fenêtre glissante étant de 1 pb;
et/ou le dispositif comprenant en outre un premier module de filtrage, couplé au module de éliminer configuré pour effectuer les opérations suivantes sur le deuxième résultat de positionnement provenant du module de éliminer:
éliminer les fragments courts ayant une longueur de connectivité inférieure à un seuil prédéterminé, et remplacer le deuxième résultat de positionnement par un résultat après suppression, la longueur de connectivité étant la longueur totale des fragments courts du deuxième résultat de positionnement qui proviennent de la séquence lue et qui sont positionnés sur différentes entrées de la bibliothèque de référence mappée à la séquence de référence;
et/ou le dispositif comprenant en outre un deuxième module de filtrage, couplé au module de éliminer et configuré pour:
déterminer, en fonction d'un résultat de positionnement de fragments courts issus de la lecture dans le deuxième résultat de positionnement, un résultat de positionnement de la lecture, et éliminer les fragments courts correspondant à la lecture dont le résultat de la détermination ne satisfait pas à une exigence prédéterminée,
dans lequel, de manière facultative, le deuxième module de filtrage est configuré pour:
attribuer un score, en fonction du résultat de positionnement des fragments courts issus de la lecture figurant dans le deuxième résultat de positionnement, du résultat de positionnement de la lecture, et éliminer la lecture lorsque son score n'est pas supérieur à une première valeur prédéfinie; ou
attribuer un score, en fonction du résultat de positionnement des fragments courts issus de la lecture figurant dans le deuxième résultat de positionnement, du résultat de positionnement de la lecture, et éliminer la lecture lorsque son score n'est pas inférieur à une deuxième valeur prédéfinie;
et/ou dans lequel le module d'extension est configuré pour:
effectuer une construction de séquence sur la base des fragments courts issus de la lecture afin d'obtenir une séquence reconstruite; et
étendre sur la base d'une partie commune de la séquence reconstruite et de la séquence de référence correspondant à la séquence reconstruite afin d'obtenir une séquence d'extension.

10. Dispositif selon la revendication 9, dans lequel la partie commune est une sous-chaîne commune; dans lequel, de manière facultative, le module d'extension est configuré pour:
trouver une sous-chaîne commune de la séquence reconstruite et de la séquence de référence correspondant à la séquence reconstruite, et déterminer la plus longue sous-chaîne commune de la séquence reconstruite et de la séquence de référence correspondant à la séquence reconstruite; et
étendre la plus longue sous-chaîne commune en fonction d'une distance d'édition afin d'obtenir une séquence d'extension;
le dispositif comprenant en outre, de manière facultative :
un module de troncature configuré pour:
tronquer la séquence d'extension à partir d'au moins une extrémité de celle-ci issue du module d'extension, calculer une proportion de sites de positionnement d'erreurs d'une séquence d'extension tronquée, et arrêter la troncature lorsque la condition suivante est satisfaite: la proportion des sites de positionnement d'erreurs de la séquence d'extension tronquée est inférieure à une troisième valeur prédéfinie;
le module de troncature étant en outre configuré, de manière facultative, pour:
i) calculer un premier taux d'erreur et un deuxième taux d'erreur; si le premier taux d'erreur est inférieur au deuxième taux d'erreur, tronquer la séquence d'extension à partir d'une première extrémité de celle-ci afin d'obtenir une séquence d'extension tronquée,
si le premier taux d'erreur est supérieur au deuxième taux d'erreur, tronquer la séquence d'extension à partir d'une deuxième extrémité de celle-ci afin d'obtenir une séquence d'extension tronquée,
dans lequel le premier taux d'erreur correspond à la proportion de sites de positionnement erronés de la séquence d'extension tronquée obtenue en tronquant la séquence d'extension à partir de sa première extrémité,
le deuxième taux d'erreur correspond à la proportion de sites de positionnement erronés de la séquence d'extension tronquée obtenue en tronquant la séquence d'extension à partir de sa deuxième extrémité;
ii) exécuter i) en remplaçant la séquence d'extension par la séquence d'extension tronquée jusqu'à ce que la proportion de sites de positionnement erronés de la séquence d'extension tronquée soit inférieure à une quatrième valeur prédéfinie ;
le module de troncature étant en outre configuré, de manière facultative, pour:
appliquer une fenêtre glissante à la séquence d'extension à partir d'au moins une extrémité de celle-ci, et calculer une proportion de sites de positionnement erronés d'une séquence de fenêtre obtenue par la fenêtre glissante,
tronquer la séquence d'extension en fonction de la proportion de sites de positionnement erronés de la séquence de fenêtre, et arrêter la troncature lorsque la condition suivante est satisfaite: la proportion de sites de positionnement erronés de la séquence de fenêtre obtenue par la fenêtre glissante est supérieure à une cinquième valeur prédéfinie; éventuellement, la fenêtre de la fenêtre glissante n'est pas supérieure à la longueur de la séquence d'extension; et
le module de troncature étant en outre configuré, de manière facultative, pour:
i) calculer un troisième taux d'erreur et un quatrième taux d'erreur ; si le troisième taux d'erreur est inférieur au quatrième taux d'erreur, tronquer la séquence d'extension à partir d'une deuxième extrémité de celle-ci pour obtenir une séquence d'extension tronquée,
si le troisième taux d'erreur est supérieur au quatrième taux d'erreur, tronquer la séquence d'extension à partir d'une première extrémité de celle-ci pour obtenir une séquence d'extension tronquée,
dans laquelle le troisième taux d'erreur correspond à la proportion de sites de positionnement d'erreur d'une séquence de fenêtre obtenue en effectuant un glissement de fenêtre sur la séquence d'extension à partir de la première extrémité de celle-ci,
le quatrième taux d'erreur est une proportion de sites de positionnement d'erreur d'une séquence de fenêtre obtenue en effectuant un glissement de fenêtre sur la séquence d'extension à partir de la deuxième extrémité de celle-ci;
ii) exécuter i) en remplaçant la séquence d'extension par la séquence d'extension tronquée jusqu'à ce que la proportion des sites de positionnement d'erreur de la séquence de fenêtre soit supérieure à la sixième valeur prédéfinie; de manière facultative, la fenêtre de glissement n'est pas supérieure à la longueur de la séquence d'extension.

11. Dispositif selon la revendication 9, dans lequel la partie commune est une sous-séquence commune; dans lequel le module d'extension est éventuellement configuré pour:
trouver une sous-séquence commune de la séquence reconstruite et de la séquence de référence correspondant à la séquence reconstruite, et déterminer une sous-séquence commune la plus longue de la séquence reconstruite et de la séquence de référence correspondant à la séquence reconstruite; et
étendre la sous-séquence commune la plus longue sur la base d'une distance d'édition afin d'obtenir une séquence d'extension;
le module de troncature étant éventuellement configuré pour:
tronquer la séquence d'extension à partir d'au moins une extrémité de la séquence d'extension provenant du module d'extension, calculer une proportion de sites de positionnement d'erreur d'une séquence d'extension tronquée, et arrêter la troncature lorsque la condition suivante est satisfaite: la proportion des sites de positionnement d'erreur de la séquence d'extension tronquée est inférieure à une troisième valeur prédéfinie;
le module de troncature étant en outre configuré, de manière facultative, pour:
i) calculer un premier taux d'erreur et un deuxième taux d'erreur; si le premier taux d'erreur est inférieur au deuxième taux d'erreur, tronquer la séquence d'extension à partir d'une première extrémité de celle-ci afin d'obtenir une séquence d'extension tronquée,
si le premier taux d'erreur est supérieur au deuxième taux d'erreur, tronquer la séquence d'extension à partir d'une deuxième extrémité de celle-ci afin d'obtenir une séquence d'extension tronquée,
dans lequel le premier taux d'erreur correspond à la proportion de sites de positionnement erronés de la séquence d'extension tronquée obtenue en tronquant la séquence d'extension à partir de sa première extrémité,
le deuxième taux d'erreur correspond à la proportion de sites de positionnement erronés de la séquence d'extension tronquée obtenue en tronquant la séquence d'extension à partir de sa deuxième extrémité ;
ii) exécuter i) en remplaçant la séquence d'extension par la séquence d'extension tronquée jusqu'à ce que la proportion de sites de positionnement erronés de la séquence d'extension tronquée soit inférieure à une quatrième valeur prédéfinie;
comprenant en outre un module de troncature configuré pour:
appliquer une fenêtre glissante à la séquence d'extension à partir d'au moins une extrémité de celle-ci, et calculer une proportion de sites de positionnement erronés d'une séquence de fenêtre obtenue par la fenêtre glissante,
tronquer la séquence d'extension en fonction de la proportion de sites de positionnement erronés de la séquence de fenêtre, et arrêter la troncature lorsque la condition suivante est satisfaite: la proportion de sites de positionnement erronés de la séquence de fenêtre obtenue par la fenêtre glissante est supérieure à une cinquième valeur prédéfinie, dans lequel, de manière facultative, la fenêtre de la fenêtre glissante n'est pas supérieure à une longueur de la séquence d'extension;
le module de troncature étant en outre configuré, de manière facultative, pour:
i) calculer un troisième taux d'erreur et un quatrième taux d'erreur; si le troisième taux d'erreur est inférieur au quatrième taux d'erreur, tronquer la séquence d'extension à partir d'une deuxième extrémité de celle-ci pour obtenir une séquence d'extension tronquée,
si le troisième taux d'erreur est supérieur au quatrième taux d'erreur, tronquer la séquence d'extension à partir d'une première extrémité de celle-ci pour obtenir une séquence d'extension tronquée,
dans laquelle le troisième taux d'erreur correspond à la proportion de sites de positionnement d'erreurs d'une séquence de fenêtre obtenue en effectuant un glissement de fenêtre sur la séquence d'extension à partir de la première extrémité de celle-ci,
le quatrième taux d'erreur est une proportion de sites de positionnement erronés d'une séquence de fenêtre obtenue en effectuant un glissement de fenêtre sur la séquence d'extension à partir de la deuxième extrémité de celle-ci;
ii) exécuter i) en remplaçant la séquence d'extension par la séquence d'extension tronquée jusqu'à ce que la proportion de sites de positionnement erronés de la séquence de fenêtre soit supérieure à la sixième valeur prédéfinie, dans laquelle, en outre, de manière facultative, la fenêtre de glissement n'est pas supérieure à la longueur de la séquence d'extension.

12. Dispositif selon l'une quelconque des revendications 9 à 11, dans lequel la troncature a une taille de 1 pb.

13. Système d'alignement, comprenant :
un dispositif d'entrée configuré pour saisir des données;
un dispositif de sortie configuré pour fournir des données;
un processeur configuré pour exécuter un programme informatique, l'exécution dudit programme informatique comprenant la mise en œuvre du procédé selon l'une quelconque des revendications 1 à 7; et
un dispositif de stockage configuré pour stocker des données, dans lequel le dispositif de stockage comprend le programme informatique.

14. Support lisible par ordinateur comprenant des instructions qui, lorsqu'elles sont exécutées par un ordinateur, amènent celui-ci à mettre en œuvre le procédé selon l'une quelconque des revendications 1 à 7.
